(19) Europäisches Patentamt / European Patent Office / Office européen des brevets

(11) **EP 3 305 342 A1**

(12) **EUROPEAN PATENT APPLICATION**
published in accordance with Art. 153(4) EPC

(43) Date of publication:
**11.04.2018 Bulletin 2018/15**

(21) Application number: **16800111.3**

(22) Date of filing: **27.05.2016**

(51) Int Cl.:
**A61L 33/00** (2006.01)

(86) International application number:
**PCT/JP2016/065665**

(87) International publication number:
**WO 2016/190407 (01.12.2016 Gazette 2016/48)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **27.05.2015 JP 2015106972**
**27.05.2015 JP 2015106976**

(71) Applicant: **Toray Industries, Inc.**
**Tokyo 103-8666 (JP)**

(72) Inventors:
• **KADOWAKI, Koji**
**Otsu-shi**
**Shiga 520-8558 (JP)**
• **SAITO, Akihiro**
**Otsu-shi**
**Shiga 520-8558 (JP)**
• **NAKANISHI, Megumi**
**Otsu-shi**
**Shiga 520-8558 (JP)**
• **FUJITA, Masaki**
**Otsu-shi**
**Shiga 520-8558 (JP)**
• **TANAHASHI, Kazuhiro**
**Otsu-shi**
**Shiga 520-8558 (JP)**

(74) Representative: **Kador & Partner**
**Corneliusstraße 15**
**80469 München (DE)**

(54) **ANTITHROMBOTIC MATERIAL**

(57) The present invention aims to provide an antithrombogenic material which has a reduced thickness of its coating material, and which maintains high antithrombogenicity for a long period. The present invention provides an antithrombogenic material comprising: a coating material containing: a cationic polymer containing, as a constituent monomer, a compound selected from the group consisting of alkyleneimines, vinylamines, allylamines, lysine, protamine, and diallyldimethylammonium chloride; and an anionic compound containing a sulfur atom and having anticoagulant activity; and a base material whose surface is coated with the coating material; wherein the cationic polymer is covalently bound to the base material; the anionic compound containing a sulfur atom and having anticoagulant activity is immobilized on the surface of the base material by ionic bonding to the cationic polymer; and the average thickness of the coating material is 15 to 400 nm.

EP 3 305 342 A1

## Description

TECHNICAL FIELD

The present invention relates to an antithrombogenic material.

BACKGROUND ART

Medical equipment and medical instruments which are brought into contact with blood (artificial kidneys, artificial lungs, artificial blood vessels, artificial valves, stents, stent-grafts, catheters, free-thrombus capture devices, angioscopes, sutures, blood circuits, tubes, cannulae, blood bags, syringes, and the like) are required to have high antithrombogenicity for prevention of functional deterioration due to blood coagulation. In methods commonly used for giving antithrombogenicity to medical equipment and medical instruments, heparin or a heparin derivative as an anticoagulant is applied or chemically bound to a surface of a base material.

As methods for binding heparin or a heparin derivative to a surface of a base material, 1) methods in which the heparin or heparin derivative is immobilized by covalent bonding to a functional group introduced to the surface of the base material; and 2) methods in which the heparin or heparin derivative is immobilized by ionic bonding to a positively charged cationic compound introduced to the surface of the base material; are mainly known.

As methods of 1), a method in which aldehyde-modified heparin prepared by oxidation by nitrous acid treatment is covalently bound to the surface of an aminated base material (Patent Document 1), a method in which aminated heparin is bound to a cationic compound polyethyleneimine (hereinafter referred to as "PEI") to allow covalent bonding to the surface of the base material to which radicals are introduced (Patent Document 2), and a method in which PEI introduced to the surface of the base material is covalently bound to heparin in the presence of a reducing agent (Patent Document 3), have been reported.

As methods of 2), methods in which, taking advantage of the fact that heparin and heparin derivatives are ionically negatively charged, heparin or a heparin derivative is bound by ionic bonding to a positively charged cationic compound have been reported (Patent Documents 4 to 7). Since, in antithrombogenic materials obtained by these methods, elution of the heparin or heparin derivative occurs with time, the strength of antithrombogenicity can be controlled by changing the amount of the heparin or heparin derivative bound and/or the elution rate thereof. Therefore, various combinations with positively charged cationic compounds have been studied.

For example, methods in which a surface of polyethylene terephthalate (hereinafter referred to as "PET") or polyamide as a base material is treated with polyamine, which is a cationic compound, by aminolysis or amide formation reaction, and heparin is bound thereto by ionic bonding, to obtain an antithrombogenic material (Patent Documents 4 to 6), and methods in which an ion complex is formed between an organic cation mixture such as a quaternary ammonium salt, or a quaternary phosphonium compound, and heparin or a heparin derivative, and the resulting ion complex is dissolved in an organic solvent, followed by applying the solution to a surface of a base material, thereby obtaining an antithrombogenic material (Patent Documents 7 and 8), have been reported. As other methods, a method in which a polymer containing a tertiary amino group is applied to a surface of a base material, and the amino group is then modified with quaternary ammonium, followed by binding heparin thereto by ionic bonding, thereby obtaining an antithrombogenic material (Patent Document 9), and methods in which PEI, which is a cationic compound, is introduced to a surface of a base material by ozone treatment or plasma treatment, and heparin is then bound thereto by ionic bonding, thereby obtaining an antithrombogenic material (Patent Documents 10 and 11), have been reported.

A method in which a compound having a negative charge is introduced to a surface of a base material by graft polymerization by carrying out plasma irradiation, and PEI, which is a polymer having a positive charge, is then introduced thereto as a linker for ionic bonding to heparin, followed by binding heparin to the PEI by ionic bonding, thereby obtaining an antithrombogenic material (Patent Document 11), a method in which ester bonds of a PET base material are hydrolyzed with sodium hydroxide, and hydroxyl groups are then oxidized with potassium permanganate into carboxyl groups, followed by introducing PEI, which is a polymer having a positively charged functional group, thereto by dehydration condensation, and binding the introduced PEI to heparin by ionic bonding, thereby obtaining an antithrombogenic material (Patent Document 12), and the like have also be reported.

On the other hand, a method in which a negatively charged, protein non-adsorptive substance such as heparin is bound to a surface of a base material to inhibit adsorption of cells to the surface has also been reported (Patent Document 13).

PRIOR ART DOCUMENTS

[Patent Documents]

[Patent Document 1] JP 4152075 B
[Patent Document 2] JP 3497612 B
[Patent Document 3] Japanese Translated PCT Patent Application Laid-open No. 10-513074
[Patent Document 4] JP 60-041947 B
[Patent Document 5] JP 60-047287 B
[Patent Document 6] WO 2014-168198
[Patent Document 7] JP 4273965 B
[Patent Document 8] JP 10-151192 A
[Patent Document 9] JP 3341503 B
[Patent Document 10] JP 3497612 B
[Patent Document 11] JP 3834602 B
[Patent Document 12] WO 2014-168198
[Patent Document 13] JP 4982752 B

SUMMARY OF THE INVENTION

PROBLEMS TO BE SOLVED BY THE INVENTION

However, in the methods disclosed in Patent Documents 1 to 3, the degree of freedom of the heparin or heparin derivative decreases due to its covalent bonding, and it is therefore difficult to obtain the anticoagulant activity required.
Patent Documents 2 to 6 describe methods in which a positively charged cationic compound such as polyamine is introduced to a surface of a base material, and heparin or a heparin derivative, which is an anionic compound having anticoagulant activity, is bound to the cationic compound by ionic bonding to achieve the immobilization. However, there is no description on an appropriate amount of the cationic compound to be introduced. In cases where the amount of the cationic compound for coating is too small, high antithrombogenicity cannot be obtained, while in cases where the amount is too large, the cationic surface exposed after elution of heparin may promote thrombus formation. Moreover, in cases where the amount of the cationic compound for coating is too large, the surface structure of the base material may be embedded, leading to deterioration of the performance as medical equipment.
In the methods disclosed in Patent Documents 7 and 8, an ionic complex containing heparin and the like is dissolved in an organic solvent, and the resulting solution is applied to a base material. However, the organic solvent used needs to be a solvent in which the ionic complex is soluble, while the base material is insoluble. In the drying process after the application, highly hydrophilic portions in the ionic complex avoid the organic solvent to cause aggregation. Since this leads to phase separation, the solution cannot be uniformly applied to the surface of the base material at present. Moreover, covalent bonding of an organic cation mixture such as a quaternary ammonium salt, or a low molecular weight compound such as a quaternary phosphonium compound, to the base material does not occur by its application alone. Therefore, in cases of use as an antithrombogenic material, elution easily occurs when it is brought into contact with a body fluid such as blood, and the elution rate of the heparin or heparin derivative cannot be controlled.
Patent Documents 9 to 11 describe methods in which a surface of a base material is coated with a cationic polymer having an amino group, and heparin is then bound to the cationic polymer by ionic bonding. However, no study has been made on an appropriate amount of the polymer to be introduced to the surface of the base material. In cases where the amount of the polymer for coating is too small, high antithrombogenicity cannot be obtained, while in cases where the amount is too large, the cationic surface exposed after elution of heparin may promote thrombus formation. Moreover, in cases where the amount of the polymer for coating is too large, the surface structure of the base material may be embedded, leading to deterioration of the performance as medical equipment.
On the other hand, as described in Patent Document 12, it is conventionally known that attachment of heparin or the like to the base material leads to a decrease in adhesiveness of cells to the surface of the base material. Thus, in cases where an antithrombogenic material using heparin or the like is used for an artificial blood vessel, stent, stent-graft, or the like, thrombosis can be prevented, but biological incorporation of the material by adhesion/growth of endothelial cells and the like may be inhibited.
The present invention aims to provide an antithrombogenic material which has a reduced thickness of its coating material, and which maintains high antithrombogenicity for a long period.
Another object of the present invention is to provide an antithrombogenic material which does not decrease adhesiveness of cells to the surface of the base material while the antithrombogenicity is maintained.

Patent Documents 11 and 12 pay attention to introduction of a polymer having a positively charged functional group which binds to heparin by ionic bonding, but do not pay attention to the quantitative relationship between positively charged functional groups and negatively charged functional groups for studying the ratio between the amount of the positively charged functional groups and the amount of the negatively charged functional groups. Thus, these documents did not find the fact that there is an optimum range of the functional-group ratio for maximum exertion of the effect of heparin.

For exertion of the effect of heparin, it is important for the cationic polymer having a positively charged functional group to form a particular structure that easily binds to heparin on the surface of the base material having a negatively charged functional group. Therefore, control of the ratio between positively charged functional groups and negatively charged functional groups contained in the base material to which heparin is to be introduced is an important factor in development of a material having high antithrombogenicity.

In view of this, another object of the present invention is to provide an antithrombogenic material which is capable of sufficiently exerting antithrombogenicity when an anionic compound containing a sulfur atom and having anticoagulant activity is eluted into blood, by introducing a cationic polymer having a positively charged functional group to a base material having a negatively charged functional group by covalent bonding, and controlling the abundance ratio between these functional groups, followed by introducing the anionic compound containing a sulfur atom and having anticoagulant activity by ionic bonding.

MEANS FOR SOLVING THE PROBLEMS

As a result of intensive study to solve the problems described above, the present inventors discovered the following inventions (1) to (9).

(1) An antithrombogenic material comprising:

a coating material containing: a cationic polymer containing, as a constituent monomer, a compound selected from the group consisting of alkyleneimines, vinylamines, allylamines, lysine, protamine, and diallyldimethylammonium chloride; and an anionic compound containing a sulfur atom and having anticoagulant activity; and a base material whose surface is coated with the coating material; wherein
the cationic polymer is covalently bound to the base material;
the anionic compound containing a sulfur atom and having anticoagulant activity is ionically bound to the cationic polymer; and
the average thickness of the coating material is 15 to 400 nm.

(2) The antithrombogenic material according to (1), wherein the total amount of the anionic compound having anticoagulant activity contained in the coating material as calculated from anti-factor Xa activity is 50 to 1000 mIU/cm$^2$.
(3) The antithrombogenic material according to (1) or (2), wherein the abundance ratio of nitrogen atoms to the abundance of total atoms as measured by X-ray photoelectron spectroscopy (XPS) on the surface is 6.5 to 9.5 atomic percent.
(4) The antithrombogenic material according to any one of (1) to (3), wherein the weight average molecular weight of the cationic polymer is 10,000 to 1,000,000.
(5) The antithrombogenic material according to any one of (1) to (4), wherein the base material is a polyester-based polymer, and the maximum stress of the antithrombogenic material is not less than 350 MPa.
(6) The antithrombogenic material according to any one of (1) to (5),
wherein
the cationic polymer has a positively charged functional group, and the base material has a negatively charged functional group; and
the abundance ratio of the positively charged functional group to the negatively charged functional group is 8.0 to 30.
(7) The antithrombogenic material according to any one of (1) to (6), wherein the ratio of the abundance of the negatively charged functional group in the base material after the covalent bonding of the cationic polymer to the abundance of the negatively charged functional group in the base material before the covalent bonding of the cationic polymer is not more than 25%.
(8) The antithrombogenic material according to any one of (1) to (7), having cellular adhesiveness.
(9) A medical tool comprising the antithrombogenic material according to any one of (1) to (8).

The present invention also provides the following inventions (10) to (13) for solving the above problems.

(10) An antithrombogenic material comprising:

an anionic compound containing a sulfur atom and having anticoagulant activity;
a polymer having a positively charged functional group; and
a base material having a negatively charged functional group;
wherein
the sulfur-containing polysaccharide is bound to the polymer by ionic bonding;
the polymer is covalently bound to the base material; and
the ratio of the abundance of the positively charged functional group to the abundance of the negatively charged functional group is 8.0 to 30.

(11) The antithrombogenic material according to (10), wherein the ratio of the abundance of the negatively charged functional group in the base material after the covalent bonding of the polymer to the abundance of the negatively charged functional group in the base material before the covalent bonding of the polymer is not more than 25%.

(12) The antithrombogenic material according to (11) or (12), wherein the weight average molecular weight of the polymer is 10,000 to 750,000.

(13) A medical tool comprising the antithrombogenic material according to any one of (10) to (12).

EFFECT OF THE INVENTION

Since the antithrombogenic material of the present invention can maintain the structure of the surface of the base material, can suppress, through a polymer covalently bound to the base material, elution of components other than the anionic compound containing a sulfur atom and having anticoagulant activity, and can exhibit anticoagulant activity for a long period, the antithrombogenic material can be preferably used for medical tools (medical equipment and medical instruments (more specifically, artificial kidneys, artificial lungs, artificial blood vessels, artificial valves, stents, stent-grafts, catheters, free-thrombus capture devices, angioscopes, sutures, blood circuits, tubes, cannulae, blood bags, syringes, and the like)) which require antithrombogenicity.

MODE FOR CARRYING OUT THE INVENTION

The antithrombogenic material of the present invention comprises:

a coating material containing: a cationic polymer containing, as a constituent monomer, a compound selected from the group consisting of alkyleneimines, vinylamines, allylamines, lysine, protamine, and diallyldimethylammonium chloride; and an anionic compound containing a sulfur atom and having anticoagulant activity; and
a base material whose surface is coated with the coating material;
wherein
the cationic polymer is covalently bound to the base material;
the anionic compound containing a sulfur atom and having anticoagulant activity is ionically bound to the cationic polymer; and
the average thickness of the coating material is 15 to 400 nm.

The following terms used in the present description are defined as described below unless otherwise specified.

The term "antithrombogenicity" means a property which prevents blood coagulation on a surface in contact with blood. For example, "antithrombogenicity" means a property which inhibits platelet aggregation, or blood coagulation which proceeds due to, for example, activation of blood coagulation factors represented by thrombin.

The term "antithrombogenic material" means a material having antithrombogenicity. The "antithrombogenic material" may be, but does not necessarily need to be, used as a material constituting medical equipment and medical instruments (artificial kidneys, artificial lungs, artificial blood vessels, artificial valves, stents, stent-grafts, catheters, free-thrombus capture devices, angioscopes, sutures, blood circuits, tubes, cannulae, blood bags, syringes, and the like). These medical equipment and medical instruments are frequently brought into contact with blood, and blood coagulation is likely to proceed on surfaces of the medical equipment and medical instruments. Therefore, antithrombogenic materials need to be used as their materials.

Among the materials constituting an antithrombogenic material, the "base material" means a substance defined below constituting a surface to be coated with the coating material. Preferred examples of the material of the base material in the present invention include, but are not limited to, polyester-based polymers, expanded porous polytetrafluoroethylene (hereinafter referred to as "ePTFE"), polyurethane, polyetherurethane, polyamide, vinyl chloride, polycarbonate, polystyrene, polyethylene, polypropylene, polymethylpentene, and polymethyl methacrylate. Among these, polyester-based polymers are preferred as the base material of the antithrombogenic material because of their versatility, and polymers containing at least an ester as a constituent monomer are more preferred. Examples of the polymers include PET, polytrimethylene terephthalate, polybutylene terephthalate, polyethylene naphthalate, and polybutylene naphthalate. Among these, PET is more preferred as the base material of the antithrombogenic material because of its versatility. The polyester-based polymer means a polymer having an ester bond in the polymer.

The negatively charged functional group in the present invention is not limited, and examples of the negatively charged functional group include a carboxyl group, sulfonate group, phosphate group, nitro group, and carbonate group.

The method for introducing the negatively charged functional group to the base material is not limited, and examples of the method include a method in which radicals are generated in the base material by, for example, ozone treatment, corona discharge, or plasma treatment, and a polymer or a low molecular weight compound having the negatively charged functional group is introduced to the base material by chemical bonding, a method in which the negatively charged functional group is introduced to a functional group (for example, hydroxyl group or amino group) contained in the base material by chemical bonding, and a method in which, in cases of a polyester-based polymer base material, the negatively charged functional group is directly formed on the base material by hydrolysis using an acid or a base or the like.

Although the negatively charged functional group introduced by the above method may be present either on the surface of the base material or in the base material, it is preferably present on at least a part of the surface of the base material, more preferably present on the entire surface of the base material, from the viewpoint of binding to the polymer having a positively charged functional group.

The "coating material" means a material with which at least a part of the surface of the base material is coated, and the coating material in the present invention contains: a cationic polymer containing, as a constituent monomer, a compound selected from the group consisting of alkyleneimines, vinylamines, allylamines, lysine, protamine, and diallyldimethylammonium chloride; and an anionic compound containing a sulfur atom and having anticoagulant activity.

The cationic polymer constituting the coating material is a cationic polymer containing, as a constituent monomer, a compound selected from the group consisting of alkyleneimines, vinylamines, allylamines, lysine, protamine, and diallyldimethylammonium chloride. Since these constituent monomers have a cationic nitrogen atom, the polymer becomes cationic. On the other hand, the compound containing a sulfur atom and having anticoagulant activity is anionic, and can therefore bind to the polymer by ionic bonding. Examples of the anionic compound containing a sulfur atom and having anticoagulant activity include heparin, heparin derivatives, dextran sulfate, polyvinyl sulfonate, and polystyrene sulfonate. Heparin and heparin derivatives are more preferred. The heparin and heparin derivatives are not limited as long as blood coagulation reaction can be inhibited therewith, and examples of the heparin and heparin derivatives include heparin which is clinically generally and widely used, unfractionated heparin, and low molecular weight heparin, as well as heparins having high affinity to antithrombin III.

Since the cationic polymer constituting the coating material has cationic properties, it may exhibit cytotoxicity. Therefore, elution of the polymer into a body fluid such as blood is not preferred. Thus, the cationic polymer constituting the coating material is covalently bound to the surface of the base material.

The positively charged functional group in the present invention is not limited, and examples of the positively charged functional group include an amino group, imino group, quaternary ammonium group, phosphonium group, and pyridinium group. The polymer having such a positively charged functional group is, for example, a polymer containing, as a constituent monomer, a compound selected from the group consisting of alkyleneimines, vinylamines, allylamines, lysine, protamine, and diallyldimethylammonium chloride.

The method for binding the polymer having a positive charge is not limited, and examples of the method include a method in which radicals are generated in the base material by a method such as ozone treatment, corona discharge, or plasma treatment, and the polymer having a positive charge is introduced to the base material surface by chemical bonding, a method in which the polymer having a positive charge is directly introduced by utilization of aminolysis reaction of the polymer having a positive charge and a base material surface such as PET by heat treatment, a method in which the polymer having a positive charge is physically adsorbed on the base material surface, and a method in which the polymer having a positive charge is chemically bound to the base material surface by irradiation with gamma ray in a state where an aqueous solution of the polymer having a positive charge is in contact with the base material

Although the positively charged functional group introduced by the above method may be present either on the surface of the base material or in the base material, it is preferably present on at least a part of the surface of the base material, more preferably present on the entire surface of the base material, from the viewpoint of binding to the anionic compound containing a sulfur atom and having anticoagulant activity.

Since the polymer having a positively charged functional group may exhibit cytotoxicity and/or the like, its elution into a body fluid such as blood is not preferred. Thus, the polymer is preferably covalently bound to the base material.

The covalent bonding herein means a chemical bond formed by sharing of an electron(s) between atoms. In the present invention, the covalent bond is a covalent bond between atoms such as carbon, nitrogen, oxygen, and/or sulfur present on the cationic polymer constituting the coating material and the surface of the base material, and may be either a single bond or a multiple bond. Examples of the type of the covalent bond include, but are not limited to, an amine bond, azide bond, amide bond, and imine bond. Among these, from the viewpoint of ease of formation of the covalent bond, stability after bonding, and the like, an amide bond is more preferred. As a result of intensive study, the present inventors discovered that, in cases where amide bonds are formed between the cationic polymer constituting the coating material and the surface of the base material, the configuration of the cationic polymer on the surface of the base material optimizes the state of ionic bonding to the anionic compound containing a sulfur atom and having anticoagulant activity, for example, heparin or a heparin derivative. Confirmation of the covalent bonds is possible by judgment of whether elution occurs by washing with a solvent that dissolves the cationic polymer.

The cationic polymer constituting the coating material may be either a homopolymer or a copolymer. In cases where the polymer is a copolymer, the copolymer may be any of a random copolymer, block copolymer, graft copolymer, and alternating copolymer. The polymer constituting the coating material is more preferably a block copolymer since, in cases of a block copolymer, stronger ionic bonding can be achieved by interaction between a block portion(s) having continuous repeat units containing nitrogen atoms, and the anionic compound containing a sulfur atom and having anticoagulant activity.

The homopolymer herein means a macromolecular compound obtained by polymerization of a single kind of constituent monomers. The copolymer herein means a macromolecular compound obtained by copolymerization of two or more kinds of monomers. The block copolymer means a copolymer having a molecular structure in which at least two kinds of polymers having different repeat units are covalently bound to each other to form a longer chain. The block means each of the "at least two kinds of polymers having different repeat units" constituting the block copolymer.

The structure of the cationic polymer may be either linear or branched. In the present invention, the polymer is preferably branched since a branched polymer can form more stable ionic bonds at multiple positions with the anionic compound containing a sulfur atom and having anticoagulant activity.

The cationic polymer has at least one functional group selected from primary to tertiary amino groups and a quaternary ammonium group. In particular, the polymer more preferably has a quaternary ammonium group rather than primary to tertiary amine groups since a quaternary ammonium group has stronger ionic interaction with the anionic compound containing a sulfur atom and having anticoagulant activity, and hence allows easier control of the elution rate of the anionic compound containing a sulfur atom and having anticoagulant activity. The ratios of different classes of amino groups in the polymer having a positively charged functional group can be measured by, for example, $^{13}$C NMR.

The carbon numbers of the three alkyl groups constituting the quaternary ammonium group are not limited. However, in cases where the carbon numbers are too large, hydrophobicity is high, and steric hindrance is enhanced, so that the anionic compound containing a sulfur atom and having anticoagulant activity cannot effectively bind to the quaternary ammonium group by ionic bonding. Moreover, in cases where the carbon number is too large, the polymer is more likely to show cytotoxicity, so that the carbon number per alkyl group bound to the nitrogen atom constituting the quaternary ammonium group is preferably 1 to 12, more preferably 2 to 6. The carbon numbers of the three alkyl groups bound to the nitrogen atom constituting the quaternary ammonium group may be either the same or different from each other.

In the present invention, a polyalkyleneimine is preferably used as the cationic polymer since the amount of the anionic compound containing a sulfur atom and having anticoagulant activity adsorbed thereto by ionic interaction is large. Examples of the polyalkyleneimine include PEI, polypropyleneimines, and polybutyleneimines, as well as alkoxylated polyalkyleneimines. Among these, PEI is more preferred.

Specific examples of the PEI include "LUPASOL" (registered trademark) (manufactured by BASF), and "EPOMIN" (registered trademark) (manufactured by Nippon Shokubai Co., Ltd.). The PEI may be a copolymer with another/other monomer(s) or a modified body as long as the effect of the present invention is not deteriorated. The modified body herein means a cationic polymer which has the same monomer repeat units constituting the polymer, but has partially undergone, for example, radical decomposition or recombination due to the later-mentioned radiation irradiation.

The constituent monomer(s) used for forming the copolymer other than alkyleneimines, vinylamines, allylamines, lysine, protamine, and diallyldimethylammonium chloride is/are not limited, and examples of the constituent monomer(s) include ethylene glycol, propylene glycol, vinylpyrrolidone, vinyl alcohol, vinylcaprolactam, vinyl acetate, styrene, methyl methacrylate, hydroxyethyl methacrylate, and siloxane. The content of the constituent monomer(s) used for forming the copolymer other than alkyleneimines, vinylamines, allylamines, lysine, protamine, and diallyldimethylammonium chloride is preferably not more than 10% by weight since ionic bonding with the anionic compound containing a sulfur atom and having anticoagulant activity is weak in cases where the content is too large.

In the present invention, in cases where the weight average molecular weight of the cationic polymer constituting the coating material is too small, and smaller than the molecular weight of the anionic compound containing a sulfur atom and having anticoagulant activity, stable ionic bonds cannot be formed on the surface of the base material, so that the antithrombogenicity of interest is less likely to be obtained. On the other hand, in cases where the weight average molecular weight of the cationic polymer is too large, the anionic compound containing a sulfur atom and having anticoagulant activity is included in the inside of the cationic polymer, so that the anionic compound is less likely to be exposed on the outermost surface of the coating material. Thus, the weight average molecular weight of the polymer constituting the coating material is preferably 10,000 to 1,500,000, more preferably 10,000 to 1,000,000. Further, for formation of stable ionic bonds on the surface of the base material, the molecular weight is preferably larger than that of the anionic compound containing a sulfur atom and having anticoagulant activity since the antithrombogenicity of interest can be more easily achieved therewith. On the other hand, for preventing the anionic compound containing a sulfur atom and having anticoagulant activity from being included in the inside of the cationic polymer, and hence for preventing the compound from being less likely to be exposed on the outermost surface of the base material, the weight average molecular weight of the cationic polymer is preferably smaller than a certain level. Thus, the weight average molecular weight of the cationic polymer constituting the coating material is especially preferably 10,000 to 750,000. The weight average molecular weight of the cationic polymer can be measured by, for example, gel permeation chromatography or the light scattering method.

In the present invention, the heparin or heparin derivative constituting the coating material may be either purified or not purified. The heparin or heparin derivative is not limited as long as blood coagulation reaction can be inhibited therewith, and examples of the heparin or heparin derivative include heparin which is clinically generally and widely used, unfractionated heparin, and low molecular weight heparin, as well as heparins having high affinity to antithrombin III. Specific examples of the heparin include "heparin sodium" (manufactured by Organon API Inc.).

In the present invention, the present inventors intensively studied to achieve maintenance of high anticoagulant activity of the anionic compound containing a sulfur atom and having anticoagulant activity for a long period while the structure of the surface of the base material is maintained and elution of components other than the anionic compound containing a sulfur atom and having anticoagulant activity is suppressed. As a result, it was discovered that there is an optimal value of the ratio of the abundance of nitrogen atoms to the abundance of total atoms as measured by XPS on the surface of the antithrombogenic material. The abundance ratio of atoms is expressed by "atomic percent". The atomic percent is the ratio of a specific kind of atoms to the abundance of total atoms, which is taken as 100, in terms of the number of atoms.

That is, in the present invention, it was discovered that there is also a preferred value of the ratio of the abundance of nitrogen atoms to the abundance of total atoms as measured by XPS on the surface of the antithrombogenic material. That is, the ratio of the abundance of nitrogen atoms to the abundance of total atoms as measured by XPS on the surface of the antithrombogenic material is preferably 6.5 to 9.5 atomic percent, more preferably 7.0 to 9.0 atomic percent, still more preferably 7.5 to 8.5 atomic percent, from the viewpoint of increasing the antithrombogenicity. In cases where the ratio of the abundance of nitrogen atoms to the abundance of total atoms is less than 6.5 atomic percent, the amount of the cationic polymer covalently bound to the surface of the base material is small, so that the structure of the surface of the base material can be maintained. However, since the coating amount of the anionic compound containing a sulfur atom and having anticoagulant activity such as heparin or a heparin derivative which binds to the surface through the cationic polymer is small in such cases, the antithrombogenicity of interest is less likely to be obtained. On the other hand, in cases where the ratio of the abundance of nitrogen atoms to the abundance of total atoms is higher than 9.5 atomic percent, the amount of the cationic polymer covalently bound to the surface of the base material is large, so that the thickness of the coating material is affected. It was also found that, although the coating amount of the anionic compound containing a sulfur atom and having anticoagulant activity bound through the cationic polymer by ionic bonding is sufficient, as elution of the compound containing a sulfur atom and having anticoagulant activity proceeds, a large amount of exposed polymer promotes thrombus formation because of its cationic properties.

In cases where the ratio of the abundance of nitrogen atoms to the abundance of total atoms is not more than 9.5 atomic percent, the coating amount of the anionic compound containing a sulfur atom and having anticoagulant activity is appropriate, so that adhesiveness of endothelial cells can be increased. For achievement of both antithrombogenicity and cellular adhesiveness, the ratio of the abundance of nitrogen atoms to the abundance of total atoms as measured by XPS on the surface of the antithrombogenic material is preferably 6.5 to 9.5 atomic percent, more preferably 7.0 to 9.0 atomic percent, still more preferably 7.5 to 8.5 atomic percent.

More specifically, the ratio of the abundance of nitrogen atoms to the abundance of total atoms on the surface of the antithrombogenic material can be determined by XPS.

[Measurement Conditions]

Apparatus: ESCALAB 220iXL (manufactured by VG Scientific)
Excitation X-ray: monochromatic AlK $\alpha$1, 2 ray (1486.6 eV)
X-ray diameter: 1 mm
X-electron escape angle: 90° (the angle of the detector with respect to the surface of the antithrombogenic material)

The surface of the antithrombogenic material herein means the portion from the measurement surface to a depth of 10 nm as detected under the measurement conditions in XPS wherein the X-electron escape angle, that is, the angle of the detector with respect to the surface of the antithrombogenic material, is 90°. In the present invention, the base material may or may not contain nitrogen atoms. In the present invention, the base material may or may not contain nitrogen atoms.

By radiating X-ray to the surface of the antithrombogenic material, and measuring the energy of photoelectrons generated therefrom, the binding energy values of bound electrons in the substance can be determined. From the binding energy values, information on the atoms on the surface of the antithrombogenic material can be obtained, and, from the energy shift of the peak at each binding energy value, information on the valence and the binding state can be obtained. In addition, by using the peak area ratio of each peak, quantification, that is, calculation of the abundance ratios of each kind of atoms, valence, and binding state, is possible.

More specifically, the N1s peak, which indicates the presence of nitrogen atoms, appears near a binding energy value of 396 eV to 403 eV. In the present invention, it was discovered that the area ratio of the N1s peak in the whole peak is preferably 6.0 to 12.0 atomic percent. The N1s peak can be split into the n1 component (near 399 eV), which is attributed to carbon-nitrogen (hereinafter referred to as "C-N") bonds; and the n2 component (near 401 to 402 eV), which is attributed to ammonium salt, C-N (structure different from n1), and/or nitrogen oxide (hereinafter referred to as "NO"). The abundance ratio of each split peak component can be calculated according to the following Equation 1. In this calculation, the ratio of the abundance of nitrogen atoms and the ratio of the abundance of each split peak component to the abundance of total atoms are rounded to one decimal place.

$$\mathrm{Split_{ratio}} = \mathrm{N1s_{ratio}} \times (\mathrm{Split_{percent}} / 100) \ldots \text{Equation 1}$$

$\mathrm{Split_{ratio}}$: Abundance ratio of each split peak component (%)
$\mathrm{N1s_{ratio}}$: Ratio of abundance of nitrogen atoms to the abundance of total atoms (%)
$\mathrm{Split_{percent}}$: Ratio of each split peak component in the N1s peak (%)

The n2 component, which is attributed to NO, obtained by splitting the N1s peak indicates the presence of quaternary ammonium groups in the present invention. It was discovered that the ratio of the n2 component in the total component of the N1s peak, that is, $\mathrm{Split_{percent}}$ (n2), is preferably 20 to 70 atomic percent, more preferably 25 to 65 atomic percent, still more preferably 30 to 60 atomic percent. In cases where $\mathrm{Split_{percent}}$ (n2) is less than 20 atomic percent, the abundance of quaternary ammonium groups is low. Therefore, the ionic interaction with the anionic compound containing a sulfur atom and having anticoagulant activity is weak, and the antithrombogenicity of interest is less likely to be obtained because of high elution rate. On the other hand, in cases where $\mathrm{Split_{percent}}$ (n2) is higher than 70 atomic percent, the ionic interaction with the anionic compound containing a sulfur atom and having anticoagulant activity is too strong. In such cases, because of a decrease in the degree of freedom due to formation of an ionic complex, it is impossible to maintain a high anticoagulant activity for a long period, and the elution rate tends be low. Because of the above reasons, the abundance ratio of the n2 component, that is, $\mathrm{Split_{ratio}}$ (n2), which is calculated according to Equation 1, is preferably 1.4 to 8.4 atomic percent, more preferably 1.8 to 7.2 atomic percent, still more preferably 2.4 to 6.0 atomic percent.

The C1s peak, which indicates the abundance of carbon atoms, appears near a binding energy value of 282 to 292 eV. The C1s peak can be mainly split into the c1 component (near 285 eV), which is attributed to carbon-hydrogen (hereinafter referred to as "CHx") bonds suggesting the presence of a saturated hydrocarbon(s) and/or the like, to carbon-carbon (hereinafter referred to as "C-C") bonds, and/or to carbon=carbon (hereinafter referred to as "C=C") bonds; the c2 component (near 286 eV), which is attributed to carbon-oxygen (hereinafter referred to as "C-O") bonds suggesting the presence of an ether(s) and/or hydroxyl groups, and/or to carbon-nitrogen (hereinafter referred to as "C-N") bonds; the c3 component (near 287 to 288 eV), which is attributed to carbon=oxygen (hereinafter referred to as "C=O") bonds suggesting the presence of carbonyl groups; the c4 component (near 288 to 289 eV), which is attributed to oxygen=carbon-oxygen (hereinafter referred to as "O=C-O") bonds suggesting the presence of ester groups and/or carboxyl groups; and the c5 component (near 290 to 292 eV), which is attributed to $\pi$-$\pi^*$ satellite peak (hereinafter referred to as "$\pi$-$\pi$") bonds suggesting the presence of a conjugated system(s) such as benzene rings. The abundance ratio of each split peak component can be calculated according to the following Equation 2. In this calculation, the ratios of the abundance of carbon atoms and the abundance of each split peak component to the abundance of total atoms are rounded to one decimal place.

$$\mathrm{Split_{ratio}} = \mathrm{C1s_{ratio}} \times (\mathrm{Split_{percent}} / 100) \ldots \text{Equation 2}$$

Split$_{ratio}$: Abundance ratio of each split peak component (%)
C1s$_{ratio}$: Ratio of the abundance of carbon atoms to the abundance of total atoms (%)
Split$_{percent}$: Ratio of each split peak component in the C1s peak (%)

The c3 component, which is attributed to C=O bonds, obtained by splitting the C1s peak indicates the presence of amide groups in the present invention. It was discovered that the ratio of the c3 component in the total component of the C1s peak in the present invention, that is, the abundance ratio of amide groups as measured by XPS on the surface of the antithrombogenic material in the present invention, is preferably not less than 2.0 atomic percent, more preferably not less than 3.0 atomic percent. In cases where the abundance ratio of the amide groups is less than 2.0 atomic percent, the number of covalent bonds due to amide bonds between the cationic polymer constituting the coating material and the surface of the base material is small, and therefore the coating amount of the coating material is small. Moreover, the configuration of the polymer on the surface of the base material adversely affects the state of ionic bonding with the anionic compound containing a sulfur atom and having anticoagulant activity. Thus, the antithrombogenicity of interest is less likely to be obtained.

In the present invention, the amount of the positively charged functional group for ionic bonding of the anionic compound containing a sulfur atom and having anticoagulant activity can be quantified by a staining method using a dye having a negatively charged functional group.

The type of the dye having a negatively charged functional group to be used is not limited, and the dye is preferably soluble in water. Examples of the dye include Orange II, methyl orange, methyl red, thymol blue, disulfine blue, lumogallion, hydroxynaphthol blue, and Coomassie brilliant blue.

A staining method using Orange II as a dye having a negatively charged functional group in the present invention is described below more specifically.

Staining was carried out using 2 mL of Orange II acetate interference solution (pH 4.0) per 4 cm2 sample area at 37°C for 1 hour, and then excessive Orange II solution was wiped off, followed by treatment with 1 mM aqueous sodium hydroxide solution at 37°C for 30 minutes to extract the dye having a negatively charged functional group ionically bound to the positively charged functional group. After neutralization of the extract with 21 mM hydrochloric acid, the absorbances at 482 nm and 550 nm are measured using an ultraviolet-visible spectrometer (U-3900, Hitachi High-Tech Science Corporation), and the difference between the absorbances at 482 nm and 550 nm is calculated. Using a calibration curve separately prepared, the amount of positively charged functional groups in the sample is quantified from the absorbance. Here, in the present invention, the quantified number of moles of Orange II was regarded as the amount of positively charged functional groups based on the assumption that 1 mol of Orange II binds to 1 mol of positively charged functional groups.

Here, when the valence of the positively charged functional group is n, and the valence of the dye having a negatively charged functional group is m, the number of positively charged functional groups can be quantified according to the following Equation 4 irrespective of the valences of the positively charged functional groups present in the base material and the dye having a negatively charged functional group.

$$Q^{+} = a^{+} \times n / m \ldots \text{Equation 4}$$

Q$^{+}$: amount of positively charged functional groups (mol)
a$^{+}$: number of moles quantified with the dye having a negatively charged functional group (mol)
n: valence (-) of the positively charged functional groups in the base material
m: valence (-) of the dye having a negatively charged functional group

In the present invention, the amount of the negatively charged functional group in the base material for ionic bonding of the anionic compound containing a sulfur atom and having anticoagulant activity can be quantified by a staining method using a dye having a positively charged functional group.

The type of the dye having a positively charged functional group to be used is not limited, and the dye is preferably soluble in water. Examples of the dye include toluidine blue O, malachite green, methylene blue, crystal violet, and methyl violet.

A staining method using toluidine blue O as a dye having a positively charged functional group in the present invention is described below more specifically.

Staining was carried out using 2 mL of toluidine blue O phosphate interference solution (pH 7.0) per 4 cm2 sample area at 37°C for 1 hour, and then excessive toluidine blue O solution was wiped off, followed by treatment with 50% (v/v) aqueous acetic acid solution at 37°C for 30 minutes to extract the dye having a positively charged functional group ionically bound to the negatively charged functional group. The extract is subjected to measurement of the absorbances at 630 nm and 750 nm using an ultraviolet-visible spectrometer (U-3900, Hitachi High-Tech Science Corporation), and the difference between the absorbances at 630 nm and 750 nm is calculated. Using a calibration curve separately prepared, the amount of negatively charged functional groups in the sample is quantified from the absorbance. Here, in the present invention, the quantified number of moles of toluidine blue O was regarded as the amount of negatively charged functional groups based on the assumption that 1 mol of toluidine blue O binds to 1 mol of negatively charged functional groups.

Here, when the valence of the negatively charged functional group is n, and the valence of the dye having a positively charged functional group is m, the number of negatively charged functional groups can be quantified according to the following Equation 5 irrespective of the valences of the negatively charged functional groups present in the base material and the dye having a positively charged functional group.

$$Q^- = a^- \times n / m \ \dots \ \text{Equation 5}$$

$Q^-$: amount of negatively charged functional groups (mol)
$a^-$: number of moles quantified with the dye having a negatively charged functional group (mol)
n: valence (-) of the positively charged functional groups in the base material
m: valence (-) of the dye having a negatively charged functional group

The measurement of the amounts of positively charged functional groups and negatively charged functional groups in the base material and the polymer for ionic bonding of the anionic compound containing a sulfur atom and having anticoagulant activity in the staining methods is preferably carried out before binding of the anionic compound containing a sulfur atom and having anticoagulant activity. However, even in cases where the anionic compound containing a sulfur atom and having anticoagulant activity is bound by ionic bonding, the measurement of the amount of positively charged functional groups and the amount of negatively charged functional groups by the staining methods is possible if sufficient washing is possible with a solvent capable of eluting the sulfur-containing polysaccharide.

In the present invention, as a result of intensive study for achievement of high anticoagulant activity of the anionic compound containing a sulfur atom and having anticoagulant activity, the present inventors discovered that there is an optimal value range of the ratio of the abundance of positively charged functional groups to the abundance of negatively charged functional groups in the antithrombogenic material.

The ratio of the abundance of positively charged functional groups to the abundance of negatively charged functional groups in the present invention is defined as the ratio between the amount of positively charged functional groups and the amount of negatively charged functional groups, wherein the amount of negatively charged functional groups is taken as 1. It is calculated according to the following Equation 6.

$$Q_{\text{ratio}} \ (\text{-}) = Q^+ / Q^- \ \dots \ \text{Equation 6}$$

$Q_{\text{ratio}}$: ratio of the abundance of positively charged functional groups to the abundance of negatively charged functional groups
$Q^+$: amount of positively charged functional groups
$Q^-$: amount of negatively charged functional groups

In the antithrombogenic material of the present invention, the ratio of the abundance of positively charged functional groups to the abundance of negatively charged functional groups is preferably higher than 1 so that the anionic compound containing a sulfur atom and having anticoagulant activity which has a negatively charged functional group can be bound by ionic bonding. On the other hand, in cases where the ratio is too high, cytotoxicity and/or the like may be produced. The ratio is therefore preferably 8.0 to 200, more preferably 8.0 to 30.

In the present invention, as a result of intensive study for achievement of high anticoagulant activity of the anionic compound containing a sulfur atom and having anticoagulant activity, the present inventors discovered that there is an optimal value range of the ratio of the abundance of negatively charged functional groups in the base material after the covalent bonding of the cationic polymer having a positively charged functional group to the abundance of negatively charged functional groups in the base material before the covalent bonding of the cationic polymer having a positively charged functional group, in the base material of the antithrombogenic material.

In the present invention, the ratio of the abundance of negatively charged functional groups in the base material after the covalent bonding of the cationic polymer having a positively charged functional group to the abundance of negatively charged functional groups in the base material before the covalent bonding of the cationic polymer having a positively charged functional group in the antithrombogenic material is defined as the ratio between the amounts of negatively charged functional groups in the base material before and after the covalent bonding of the cationic polymer having a positively charged functional group, wherein the amount of negatively charged functional groups in the base material before the covalent bonding of the polymer having a positively charged functional group is taken as 1. It is calculated according to the following Equation 7.

$$Q^-_{residue}\,(\%) = (Q^-_{after} / Q^-_{before}) \times 100 \ \dots \text{ Equation 7}$$

$Q^-_{residue}$: ratio of the abundance of negatively charged functional groups in the base material after the covalent bonding of the cationic polymer having a positively charged functional group to the abundance of negatively charged functional groups in the base material before the covalent bonding of the cationic polymer having a positively charged functional group

$Q^-_{after}$: amount of negatively charged functional groups in the base material after the covalent bonding of the cationic polymer having a positively charged functional group

$Q^-_{before}$: amount of negatively charged functional groups in the base material before the covalent bonding of the cationic polymer having a positively charged functional group

In the antithrombogenic material of the present invention, stable covalent bonding of the cationic polymer having a positively charged functional group can be achieved by consumption of a larger number of negatively charged functional groups. Accordingly, the ratio of the abundance of negatively charged functional groups in the base material after the covalent bonding of the cationic polymer having a positively charged functional group to the abundance of negatively charged functional groups in the base material before the covalent bonding of the cationic polymer having a positively charged functional group in the antithrombogenic material is preferably small. The ratio is more preferably not more than 25%, still more preferably not more than 20%.

The antithrombogenic material of the present invention can be favorably used for medical equipment and medical instruments (artificial kidneys, artificial lungs, artificial blood vessels, artificial valves, stents, stent-grafts, catheters, free-thrombus capture devices, angioscopes, sutures, blood circuits, tubes, cannulae, blood bags, syringes, and the like). The antithrombogenic material of the present invention is especially preferably used as a material for free-thrombus capture devices and artificial blood vessels.

In cases where the antithrombogenic material of the present invention is used for a free-thrombus capture device, it is preferred to use the antithrombogenic material of the present invention for all constituents of the free-thrombus capture device. Since a porous material, which is s constituent for capturing free thrombi, requires highest antithrombogenicity, at least the porous material as the base material may be coated with the coating material. Examples of the porous material as the base material include, but are not limited to, porous membranes and meshes. Meshes are preferred since they have better uniformity of pores or apertures. Preferred examples of the material of the porous material include, but are not limited to, metals such as nickel-titanium alloy, and polyurethanes and polyesters. PET, which is a polyester, is more preferably used.

From the viewpoint of increasing the accuracy of capturing free thrombi, in cases where the mesh as the material is PET, the single fiber diameter of the fibers constituting the mesh is preferably 10 to 50 $\mu$m, more preferably 20 to 40 $\mu$m. The mesh aperture is preferably 10 to 200 $\mu$m, more preferably 50 to 150 $\mu$m.

In cases where the antithrombogenic material of the present invention is used for an artificial blood vessel, it is preferred to use the antithrombogenic material of the present invention for all constituents of the artificial blood vessel. Since the inner surface of the artificial blood vessel is brought into contact with blood, and therefore requires highest antithrombogenicity, at least the inner surface of the artificial blood vessel as the base material may be coated with the coating material. Preferred examples of the material constituting the inner surface of the artificial blood vessel as the base material include, but are not limited to, fabric structures composed of warp and weft yarns constituted by monofilaments or multifilaments. Preferred examples of the material of the base material include, but are not limited to, nylons and polyester-based polymers, and ePTFE. PET, which is a polyester-based polymer, is more preferably used.

From the viewpoint of achieving favorable flexibility of the artificial blood vessel, in cases where the mesh as the material is PET, monofilaments and multifilaments having a single fiber diameter of not more than 15 $\mu$m are preferred; monofilaments and multifilaments having a single fiber diameter of not more than 10 $\mu$m are more preferred; and monofilaments and multifilaments having a single fiber diameter of not more than 5 $\mu$m are still more preferred.

In cases of a conventional antithrombogenic material, coating of the mesh as the base material with a coating material may cause destruction of the microstructure of the mesh, apertures, leading to a decrease in the accuracy of capturing thrombi. Moreover, destruction of the microstructure of the inner surface of the artificial blood vessel, that is, the fabric structure composed of warp and weft yarns, may affect blood flow and/or the like to promote thrombus formation.

However, in the antithrombogenic material of the present invention, in cases where the average thickness of the coating material is 15 to 400 nm, high antithrombogenicity can be maintained for a long period without destroying the microstructure of apertures of the mesh used for a free-thrombus capture device, or the microstructure of the fabric structure used for the inner surface of an artificial blood vessel.

In cases where the average thickness of the coating material covering the base material is too large, the surface structure of the base material may be destroyed, so that the performance of the medical equipment may be affected. On the other hand, in cases where the coating material is too thin, the antithrombogenicity of interest cannot be obtained. In cases where the average thickness of the coating material is too large, the cationic surface exposed after elution of the compound having anticoagulant activity promotes thrombus formation. Thus, the average thickness of the coating material is preferably not less than 15 nm, more preferably not less than 20 nm. On the other hand, the average thickness is preferably not more than 400 nm, more preferably not more than 200 nm, still more preferably not more than 100 nm. The average thickness herein means the thickness in which atoms derived from the coating material can be observed using the later-mentioned scanning transmission electron microscope (hereinafter referred to as "STEM"). The average thickness is expressed as the average of the values obtained at at least three positions.

Methods for producing the antithrombogenic material of the present invention are described below. For example, in preparation of fibers constituting the mesh as the base material of a free-thrombus capture device, or in preparation of fibers constituting the fabric structure as the base material of an artificial blood vessel, coating with the coating material may be carried out by adding the base material of interest to a solution which contains a cationic polymer containing, as a constituent monomer, a compound selected from the group consisting of alkyleneimines, vinylamines, allylamines, lysine, protamine, and diallyldimethylammonium chloride, and the anionic compound containing a sulfur atom and having anticoagulant activity. Alternatively, the surface of the base material may be coated with the coating material after entirely or partially reacting the cationic polymer with the anionic compound containing a sulfur atom and having anticoagulant activity.

In particular, from the viewpoint of efficiently allowing the surface of the base material to exhibit antithrombogenicity, a method in which the cationic polymer containing, as a constituent monomer, a compound selected from the group consisting of alkyleneimines, vinylamines, allylamines, lysine, protamine, and diallyldimethylammonium chloride, is covalently bound to the surface of the base material in a first coating step, and then the anionic compound containing a sulfur atom and having anticoagulant activity is bound to the polymer by ionic bonding in a second coating step, is more preferred.

In cases where the polymer contains a primary to tertiary amino group(s), a step of modifying the polymer with quaternary ammonium may be included after the first coating step, in order to increase ionic interaction with the anionic compound containing a sulfur atom and having anticoagulant activity, and to enable easy control of the elution rate of the anionic compound containing a sulfur atom and having anticoagulant activity.

The following is a production method in a case where the method in which the cationic polymer containing, as a constituent monomer, a compound selected from the group consisting of alkyleneimines, vinylamines, allylamines, lysine, protamine, and diallyldimethylammonium chloride, is covalently bound to the surface of the base material in a first coating step, and then the anionic compound containing a sulfur atom and having anticoagulant activity is bound to the polymer by ionic bonding in a second coating step, is used.

The method for covalent bonding of the cationic polymer to the surface of the base material is not limited. In cases where the base material has a functional group(s) (for example, hydroxyl, thiol, amino, carboxyl, aldehyde, isocyanate, and/or thioisocyanate), the cationic polymer may be covalently bound thereto by chemical reaction. For example, in cases where the surface of the base material has a carboxyl group and/or the like, a cationic polymer having a hydroxyl group, thiol group, amino group, and/or the like may be covalently bound to the surface of the base material. Examples of the method of the covalent bonding include a method in which a compound having a hydroxyl group, thiol group, amino group, and/or the like is covalently bound to the cationic polymer, and the resulting polymer is covalently bound to the surface of the base material having a carboxyl group and/or the like.

In cases where the base material does not have a functional group, examples of the method include a method in which the surface of the base material is treated with plasma, corona, or the like, followed by covalent bonding of the cationic polymer thereto, and a method in which radiation irradiation is performed to cause generation of radicals on the surface of the base material and the polymer, and covalent bonding between the surface of the base material and the cationic polymer is achieved by recombination reaction of the radicals. As the radiation, $\gamma$-ray or electron beam is commonly employed. In cases where $\gamma$-ray is employed, the amount of the $\gamma$-ray source is preferably 2,500,000 to 10,000,000 Ci, more preferably 3,000,000 to 7,500,000 Ci. In cases where electron beam is employed, the accelerating voltage of the electron beam is preferably not less than 5 MeV, more preferably not less than 10 MeV. The radiation dose is preferably 1 to 50 kGy, more preferably 5 to 35 kGy. The irradiation temperature is preferably 10 to 60°C, more preferably 20 to 50°C.

In cases of the method in which radiation irradiation is carried out for covalent bonding, an antioxidant may be used for controlling the amount of radicals generated. The antioxidant herein means a molecule which tends to give electrons to other molecules. Examples of the antioxidant to be used include, but are not limited to, water-soluble vitamins such as vitamin C; polyphenols; alcohols such as methanol, ethanol, propanol, ethylene glycol, propylene glycol, and glycerin; sugars such as glucose, galactose, mannose, and trehalose; inorganic salts such as sodium hydrosulfite, sodium pyrosulfite, and sodium dithionate; uric acid; cysteine; glutathione; and buffers such as bis(2-hydroxyethyl)iminotris(hydroxymethyl)methane (hereinafter referred to as "Bis-Tris"). From the viewpoint of ease of handling, residual performance, and the like, methanol, ethanol, propylene glycol, and Bis-Tris are preferred. Propylene glycol and Bis-Tris are more preferred. These antioxidants may be used individually, or may be used as a mixture of two or more of these. The antioxidant is preferably added to an aqueous solution.

In cases where a polyester-based polymer is used as the material of the base material, the cationic polymer may be brought into contact therewith under heat to allow covalent bonding by aminolysis reaction, although the method of covalent bonding is not limited thereto. Alternatively, ester bonds on the surface of the base material may be hydrolyzed by acid or alkali treatment, and carboxyl groups generated on the surface of the base material may be covalently bound to amino groups in the cationic polymer by condensation reaction. In these methods, the reaction may be carried out by bringing the cationic polymer into contact with the surface of the base material, or by bringing a solution of the cationic polymer in a solvent into contact with the surface of the base material. Preferred examples of the solvent include water and alcohols. From the viewpoint of ease of handling, residual performance, and the like, water is especially preferred. Alternatively, the monomers constituting the cationic polymer may be polymerized in a state where the monomers are in contact with the surface of the base material, and the reaction may then be carried out to achieve covalent bonding.

Examples of the means for the heating include, but are not limited to, electric heating, microwave heating, and far-infrared heating. In cases where the cationic polymer is to be covalently bound to a polyester-based polymer base material by aminolysis reaction, the aminolysis reaction of the cationic polymer with the polyester-based polymer base material is less likely to proceed at a low heating temperature. The heating temperature is therefore preferably not less than a temperature near the glass transition temperature. On the other hand, in cases where the heating temperature is too high, the aminolysis reaction sufficiently proceeds, but the skeletal structure of the polyester-based polymer base material is likely to be destroyed. The heating temperature is therefore preferably not more than the melting point.

In the present invention, the surface of the polyester-based polymer base material is more preferably hydrolyzed and oxidized before the first coating step. By the hydrolysis and oxidation of the surface of the polyester-based polymer base material, hydrolysis and oxidation of ester bonds occur, so that the coating material becomes more likely to be bound thereto. More specifically, as the method of the hydrolysis and oxidation, a method in which treatment is carried out using an acid or an alkali, as well as an oxidizing agent, is preferably used. The method of the hydrolysis and oxidation may be treatment with only an acid or an alkali. However, from the viewpoint of preventing contamination with hydroxyl groups and carboxyl groups produced by hydrolysis of ester bonds to allow the condensation reaction with amino groups of the cationic polymer to proceed efficiently, and from the viewpoint of reducing the existing hydroxyl groups to prevent activation of complement upon contact with blood, a method by treatment with an acid or an alkali, as well as an oxidizing agent, is especially preferably used for increasing the coating amount of the cationic polymer to increase the antithrombogenicity.

In cases where ester bonds in the base material are hydrolyzed using an acid or a base, the produced hydroxyl groups are preferably oxidized into carboxylic acid. This is because the presence of hydroxyl groups is not preferred for the antithrombogenic material since they are known to tend to activate complement when they are in contact with blood.

In the present invention, the step of hydrolyzing and oxidizing ester bonds on the surface of the polyester-based polymer base material using an acid or an alkali, as well as an oxidizing agent, is preferably a method in which the treatment is carried out with a combination of an acid and an oxidizing agent. The treatment using an acid and an oxidizing agent may be carried out after treating the surface of the base material using an alkali.

Examples of the type of the acid used include, but are not limited to, inorganic acids such as hydrochloric acid, hydrobromic acid, hydroiodic acid, hypochlorous acid, chlorous acid, perchloric acid, sulfuric acid, fluorosulfonic acid, nitric acid, phosphoric acid, hexafluoroantimonic acid, tetrafluoroboric acid, chromic acid, and boric acid; sulfonic acids such as methanesulfonic acid, ethanesulfonic acid, benzenesulfonic acid, p-toluenesulfonic acid, trifluoromethanesulfonic acid, and sodium polystyrene sulfonate; carboxylic acids such as acetic acid, citric acid, formic acid, gluconic acid, lactic acid, oxalic acid, and tartaric acid; vinyl carboxylic acids such as ascorbic acid and Meldrum's acid; and nucleic acids such as deoxyribonucleic acid and ribonucleic acid. Among these, hydrochloric acid, sulfuric acid, and the like are more preferred from the viewpoint of, for example, ease of handling.

In the present invention, it was found that, in the step of hydrolyzing and oxidizing ester bonds on the surface of the polyester-based polymer base material before the first coating step, the concentration of the acid for the treatment is important. For example, in cases where sulfuric acid is used, the sulfuric acid concentration is preferably 0.1 to 2.0 M, more preferably 0.1 to 1.0 M. In cases where the sulfuric acid concentration is too low, introduction, to the surface of the polyester-based polymer base material, of carboxyl groups in an amount sufficient for the condensation reaction with amino groups in the cationic polymer is impossible, while in cases where the sulfuric acid concentration is too high, the base material becomes fragile, and therefore the performance of the medical equipment may be affected.

Examples of the type of the base used include, but are not limited to, hydroxides of alkali metals such as lithium hydroxide, sodium hydroxide, potassium hydroxide, rubidium hydroxide, and cesium hydroxide; hydroxides of tetraalkylammonium such as tetramethylammonium hydroxide and tetraethylammonium hydroxide; hydroxides of alkaline earth metals such as calcium hydroxide, strontium hydroxide, barium hydroxide, europium hydroxide, and thallium hydroxide; guanidine compounds; hydroxides of ammine complexes such as diammine silver (I) hydroxide and tetraammine copper (II) hydroxide; trimethylsulfonium hydroxide; and diphenyliodonium hydroxide. Among these, lithium hydroxide, sodium hydroxide, potassium hydroxide, and the like are more preferred from the viewpoint of, for example, ease of handling.

In the present invention, it was found that, in the step of hydrolyzing and oxidizing ester bonds on the surface of the polyester-based polymer base material before the first coating step, the concentration of the base for the treatment is important. For example, in cases where sodium hydroxide is used, the sodium hydroxide concentration is preferably 0.5 to 2.0%, more preferably 0.5 to 1.0%. In cases where the sodium hydroxide concentration is too low, introduction, to the surface of the polyester-based polymer base material, of carboxyl groups in an amount sufficient for the condensation reaction with amino groups in the cationic polymer is impossible, while in cases where the sodium hydroxide concentration is too high, the base material becomes fragile, and therefore the performance of the medical equipment may be affected.

Examples of the type of the oxidizing agent used include, but are not limited to, potassium nitrate; hypochlorous acid; chlorous acid; perchloric acid; halogens such as fluorine, chlorine, bromine, and iodine; permanganates such as potassium permanganate, sodium permanganate trihydrate, ammonium permanganate, silver permanganate, zinc permanganate hexahydrate, magnesium permanganate, calcium permanganate, and barium permanganate; ceric ammonium nitrate; chromic acid; dichromic acid; peroxides such as hydrogen peroxide solution; Tollens' reagent; and sulfur dioxide. Among these, permanganates are more preferred from the viewpoint of, for example, their strength as oxidizing agents and favorable prevention of deterioration of the antithrombogenic material.

In the present invention, it was found that, in the step of hydrolyzing and oxidizing ester bonds on the surface of the polyester-based polymer base material before the first coating step, the concentration of the oxidizing agent for the treatment is important. For example, in cases where potassium permanganate is used, the potassium permanganate concentration is preferably 0.5 to 4.0%, more preferably 1.0 to 3.0%. In cases where the potassium permanganate concentration is too low, introduction, to the surface of the polyester-based polymer base material, of carboxyl groups in an amount sufficient for the condensation reaction with amino groups in the cationic polymer is impossible, while in cases where the potassium permanganate concentration is too high, the base material becomes fragile, and therefore the performance of the medical equipment may be affected.

Examples of the method for covalently binding the cationic polymer to the surface of the polyester-based polymer base material include a method in which condensation reaction is carried out using a dehydration-condensation agent or the like.

Examples of the type of the dehydration-condensation agent used include, but are not limited to, carbodiimide compounds such as N,N'-dicyclohexyl carbodiimide, N,N'-diisopropyl-carbodiimide, 1-ether-3-(3-dimethylaminopropyl)carbodiimide, 1-ether-3-(3-dimethylaminopropyl)carbodiimide hydrochloride (hereinafter referred to as "EDC"), 1,3-bis(2,2-dimethyl-1,3-dioxolan-4-ylmethyl)carbodiimide, N-{3-(dimethylamino)propyl-}-N'-ethylcarbodiimide, N-{3-(dimethylamino)propyl-}-N'-ethylcarbodiimide methiodide, N-tert-butyl-N'-ethylcarbodiimide, N-cyclohexyl-N'-(2-morpholinoethyl)carbodiimide meso-p-toluenesulfonate, N,N'-di-tert-butylcarbodiimide, and N,N'-di-p-tricarbodiimide; and triazine compounds such as 4(-4,6-dimethoxy-1,3,5-triazin-2-yl)-4-methylmorpholinium chloride n-hydrate (hereinafter referred to as "DMT-MM").

The dehydration-condensation agent may be used together with a dehydration-condensation promoter. Examples of the dehydration-condensation promoter used include, but are not limited to, pyridine, 4-dimethylaminopyridine (hereinafter referred to as "DMAP"), triethylamine, isopropylamine, 1-hydroxybenzotriazole, and N-hydroxysuccinimide.

The cationic polymer, polyester-based polymer, dehydration-condensation agent, and dehydration-condensation promoter may be prepared as a mixed aqueous solution to be used for the reaction, or may be sequentially added to perform the reaction.

In cases where ePTFE is used as a material of the base material, a method in which the surface of the base material is functionalized using plasma or corona may be used, although the method is not limited thereto. Alternatively, a method in which a fluorocarbon-resin surface treatment agent or the like is used for extraction of fluorine atoms present on the surface of the base material, and hydroxyl groups, carboxyl groups, carbonyl groups, and/or the like, for example, are formed by reaction with oxygen, hydrogen, water vapor, and/or the like in the air, may be used.

In the same manner as in the cases of the polyester-based polymer base material described above, a first coating step of covalently binding the cationic polymer to the surface of the ePTFE base material may be carried out.

In cases where the cationic polymer contains a primary to tertiary amino group(s), a step of modifying the cationic polymer with quaternary ammonium may be included in order to increase ionic interaction with the anionic compound containing a sulfur atom and having anticoagulant activity, and to enable easy control of the elution rate of the anionic compound containing a sulfur atom and having anticoagulant activity.

## EP 3 305 342 A1

In terms of the method for modification of the cationic polymer with quaternary ammonium, the cationic polymer may be modified with quaternary ammonium before covalent bonding of the cationic polymer to the surface of the base material, or the cationic polymer may be modified with quaternary ammonium after covalent bonding of the cationic polymer to the surface of the base material. From the viewpoint of increasing the ionic interaction between the cationic polymer and the anionic compound containing a sulfur atom and having anticoagulant activity, quaternary ammonium groups contained in the cationic polymer are preferably present on the outermost surface of the coating material. Thus, the modification with quaternary ammonium is preferably carried out after covalent bonding of the polymer to the surface of the base material. More specifically, after covalently binding the cationic polymer to the surface of the base material, an alkyl halide compound such as ether chloride or ethyl bromide, or a glycidyl-containing quaternary ammonium salt, may be directly brought into contact with the polymer, or may be brought into contact with the polymer after dissolving it in an aqueous solution or an organic solvent.

The second coating step of binding the anionic compound containing a sulfur atom and having anticoagulant activity to the cationic polymer by ionic bonding is not limited, and a method in which an aqueous solution of the compound is brought into contact with the cationic polymer is preferred.

In the present invention, the level of hydrolysis and oxidation of ester bonds on the surface of the polyester-based polymer base material is evaluated using as a standard the maximum stress of the base material coated with the coating material. The maximum stress of the base material can be calculated from, for example, the maximum load evaluated using a tensile tester and the cross-sectional area of the base material as shown by the following Equation 8.

$$\text{Maximum stress of the base material coated with the coating material (MPa)}$$

$$= \text{maximum load (N)} \, / \, \text{cross-sectional area of the base material (mm}^2) \ldots \text{Equation 8}$$

[Measurement Conditions]

Apparatus: tensile tester Tensilon RTE-1210 (manufactured by Orientec Co., Ltd.)
Load cell: 500 N
Initial sample length: 10 mm
Pulling rate: 5 mm/minute

In the present invention, in cases where the maximum stress of the polyester-based polymer base material coated with the coating material is not less than 350 MPa, the possibility that the performance of the medical equipment is affected is very small, which is preferred.

In cases where the ratio of the maximum stress of the base material coated with the coating material to the maximum stress of the untreated base material (which has not been subjected to the step of hydrolysis and oxidation of ester bonds on the surface) is not less than 80%, the possibility that the performance of the medical equipment is affected is very small, which is preferred.

In the present invention, the anti-factor Xa activity on the surface of the antithrombogenic material was measured. The anti-factor Xa activity herein is an index indicating the degree of inhibition of the activity of factor Xa, which promotes conversion of prothrombin to thrombin. For example, in cases where the anionic compound containing a sulfur atom and having anticoagulant activity in the antithrombogenic material is heparin or a heparin derivative, its surface amount can be known based on the unit of anti-factor Xa activity. For the measurement, "Test Team (registered trademark) Heparin S" (manufactured by Sekisui Medical Co., Ltd.) was used. In cases where the anti-factor Xa activity is too low, the surface amount of the heparin or heparin derivative in the antithrombogenic material is small, and the antithrombogenicity of interest is less likely to be obtained. That is, the anti-factor Xa activity is preferably not less than 40 mIU/cm$^2$, more preferably not less than 50 mIU/cm$^2$, still more preferably not less than 60 mIU/cm$^2$. The surface amount estimated based on the anti-factor Xa activity herein means a value measured after 30 minutes of immersion in physiological saline at 37°C.

In the antithrombogenic material of the present invention, irrespective of the fact that the total supported amount of the heparin or heparin derivative with which the surface of the base material is coated as estimated based on the anti-factor Xa activity is small, the initial surface amount of the heparin or heparin derivative after the 30 minutes of immersion in physiological saline is large. The total supported amount herein means the sum of the total elution amount and the surface amount remaining on the surface of the antithrombogenic material measured based on the anti-factor Xa activity. In cases where the total coating amount is too large, the microstructure of the surface of the base material is destroyed, while in cases where the total coating amount is too small, the antithrombogenicity of interest is less likely to be obtained.

Moreover, in cases where the total supported amount is too large, the cationic polymer is likely to be exposed after elution of heparin. That is, the total supported amount on the surface of the antithrombogenic material as estimated based on the anti-factor Xa activity is preferably 50 to 1000 $mIU/cm^2$, more preferably 60 to 1000 $mIU/cm^2$, still more preferably 60 to 800 $mIU/cm^2$. The elution amount herein means the amount eluted into human blood plasma after immersion in the human blood plasma at 37°C for 24 hours. For the measurement, "Test Team (registered trademark) Heparin S" (manufactured by Sekisui Medical Co., Ltd.) was used.

As a result of an animal experiment using the antithrombogenic material of the present invention, it was discovered that the anti-factor Xa activity after blood plasma washing is important for achievement of both antithrombogenicity and cellular adhesiveness. More specifically, preferably, the anti-factor Xa activity remaining on the surface of the antithrombogenic material after immersion in blood plasma for 6 hours is 1 to 40 $mIU/cm^2$, and the initial surface activity after immersion in physiological saline for 30 minutes is not less than 40 $mIU/cm^2$. More preferably, the anti-factor Xa activity remaining on the surface of the antithrombogenic material after immersion in blood plasma for 6 hours is 5 to 40 $mIU/cm^2$, and the initial surface activity after immersion in physiological saline for 30 minutes is not less than 50 $mIU/cm^2$, still more preferably not less than 80 $mIU/cm^2$.

In the present invention, the anti-factor Xa activity on the surface of the antithrombogenic material was measured. The anti-factor Xa activity herein is an index indicating the degree of inhibition of the activity of factor Xa, which promotes conversion of prothrombin to thrombin. For example, in cases where the anionic compound containing a sulfur atom and having anticoagulant activity in the antithrombogenic material is heparin or a heparin derivative, its surface amount can be known based on the unit of anti-factor Xa activity. For the measurement, "Test Team (registered trademark) Heparin S" (manufactured by Sekisui Medical Co., Ltd.) was used. In cases where the surface amount of the heparin or heparin derivative in the antithrombogenic material is too small, the antithrombogenicity of interest is less likely to be obtained. Thus, the anti-factor Xa activity is preferably at not less than a certain level. On the other hand, there may be cases where, while the surface amount of the heparin or heparin derivative is sufficient for achievement of the antithrombogenicity of interest, an increase in the thickness of the heparin or heparin derivative present on the surface of the base material leads to difficulty in maintenance of the microstructure of the surface of the base material. Therefore, the anti-factor Xa activity is preferably not too high. That is, the anti-factor Xa activity is preferably 40 $mIU/cm^2$, more preferably 80 $mIU/cm^2$. The surface amount estimated based on the anti-factor Xa activity herein means a value measured after 30 minutes of immersion in physiological saline.

In the present invention, elution of the anionic compound containing a sulfur atom and having anticoagulant activity proceeds as the antithrombogenic material is continuously used. In this process, the exposed polymer having a positively charged functional group might exhibit hemolytic toxicity. As an index indicating the hemolytic toxicity, the hemolysis rate calculated according to the following Equation 9 was used. Hemolytic toxicity is ranked into different grades based on the value of the hemolysis rate as shown in Table 1, according to the hemolytic toxicity test described in a guideline published by Ministry of Health, Labour and Welfare, "Basic Principles of Biological Safety Evaluation Required for Application for Approval to Market Medical Devices". The hemolytic toxicity in the present invention is preferably ranked into the "nonhemolytic" or "mildly hemolytic" grade, more preferably ranked into the "nonhemolytic" grade.

$$\text{Hemolysis rate (\%)} = [(At - An) / (Ap - An)] \times 100 \ldots \text{Equation 9}$$

At: absorbance of the sample
An: absorbance of the negative control
Ap: absorbance of the positive control

[Table 1]

| Hemolysis rate (%) | Grade |
| --- | --- |
| Hemolysis rate $\leqq$ 2 | Nonhemolytic |
| 2 < Hemolysis rate $\leqq$ 10 | Mildly hemolytic |
| 10 < Hemolysis rate $\leqq$ 20 | Moderately hemolytic |
| 20 < Hemolysis rate $\leqq$ 40 | Strongly hemolytic |
| 40 < Hemolysis rate | Very strongly hemolytic |

In the antithrombogenic material of the present invention, the coating material constituted by the cationic polymer, anionic compound containing a sulfur atom and having anticoagulant activity, and the like is preferably present not only as a coating on the interface of the base material, but also in the depth direction from the interface of the base material.

More specifically, whether or not the coating material is present in the depth direction from the interface of the base material can be confirmed by combination of, for example, a STEM and XPS. A STEM has detectors such as an energy dispersive X-ray spectrometer (hereinafter referred to as "EDX") and an electron energy-loss spectrometer (hereinafter referred to as "EELS"). Measurement conditions for the STEM are as follows.

[Measurement Conditions]

Apparatus: field emission transmission electron microscope JEM-2100F (manufactured by JEOL Ltd.)
EELS detector: GIF Tridiem (manufactured by GATAN, Inc.)
EDX detector: JED-2300T (manufactured by JEOL Ltd.)
Image acquisition: Digital Micrograph (manufactured by GATAN, Inc.)
Sample preparation: ultrathin sectioning (suspension using a copper microgrid; use of an acrylic resin as an embedding resin)
Acceleration voltage: 200 kV
Beam diameter: 0.7 nm diameter
Energy resolution: about 1.0 eV FWHM

The surface of the antithrombogenic material herein means the portion from the measurement surface to a depth of 10 nm as measured by XPS, and the interface of the antithrombogenic material herein means the border with the acrylic resin in which the antithrombogenic material is embedded in the sample preparation before the measurement using the STEM.

Whether or not the coating material is present in the depth direction from the interface of the base material can be judged by, for example, measurement using the STEM. It is possible by carrying out observation of atoms derived from the coating material which is the polymer and the anionic compound containing a sulfur atom and having anticoagulant activity, from the interface toward the depth direction of the antithrombogenic material, and finding atoms derived from the coating material at a position deeper than a position where atoms derived from the base material are found. For example, in cases where the base material is a polyester-based polymer or ePTFE, the presence of the coating material can be confirmed by finding nitrogen atoms derived from the polymer and/or sulfur atoms derived from the anionic compound containing a sulfur atom and having anticoagulant activity, at a position deeper than a position where oxygen atoms, fluorine atoms, and/or the like derived from the base material are found. The interface of the base material herein means the position in the depth direction where atoms derived from the base material are found. Here, the presence of atoms is judged based on whether a peak intensity derived from the atoms can be found in a spectrum obtained by STEM measurement after subtraction of the background.

In the present invention, atoms derived from the coating material, which is the polymer and the anionic compound containing a sulfur atom and having anticoagulant activity, are present at positions more distant from the interface of the antithrombogenic material in the depth direction from the position of the interface of the base material. More specifically, nitrogen atoms and sulfur atoms are preferably present to a depth of at least 10 to 100 nm, more preferably present to a depth of 20 to 90 nm, from the interface of the base material. In the present invention, in cases where the coating material is present to a depth of at least 10 to 100 nm from the interface of the base material, preferred values of the amount of the anionic compound containing a sulfur atom and having anticoagulant activity eluted and the elution rate of the compound can be obtained, so that high antithrombogenicity is likely to be maintained for a long period. In cases where the coating material is present to a depth of only less than 10 nm, elution of the anionic compound containing a sulfur atom and having anticoagulant activity is likely to occur. On the other hand, in cases where the coating material is present to a depth of more than 100 nm, the amount of the compound eluted and the elution rate become favorable, but deterioration due to hydrolysis of the polyester-based polymer caused by the acid or the alkali, and the oxidizing agent, is likely to occur, so that mechanical properties of the base material such as the tensile strength are likely to be deteriorated. Accordingly, in the present invention, in cases of a base material which is not a porous material having pores, the coating material is preferably bound to a depth of 10 to 100 nm.

EXAMPLES

The present invention is described below in detail by way of Examples and Comparative Examples. However, the present invention is not limited thereto.

(Example 1)

A PET mesh (diameter, 27 μm; interfiber distance, 100 μm) as a base material was immersed in an aqueous solution of 3.0 wt% potassium permanganate (manufactured by Wako Pure Chemical Industries, Ltd.) and 0.6 mol/L sulfuric acid (manufactured by Wako Pure Chemical Industries, Ltd.), and the reaction was allowed to proceed at 60°C for 3 hours, thereby hydrolyzing and oxidizing the PET mesh (hydrolysis/oxidation step). The aqueous solution after the reaction was removed, and the mesh was washed with hydrochloric acid (manufactured by Wako Pure Chemical Industries, Ltd.) and distilled water.

Subsequently, the PET mesh was immersed in an aqueous solution of 0.5 wt% DMT-MM (manufactured by Wako Pure Chemical Industries, Ltd.) and 5.0 wt% PEI (average molecular weight, about 10,000; manufactured by Wako Pure Chemical Industries, Ltd.), which is a part of the coating material, and the reaction was allowed to proceed at 30°C for 2 hours, thereby covalently binding PEI to the PET mesh by condensation reaction (first coating step). The aqueous solution after the reaction was removed, and the mesh was washed with distilled water.

The PET mesh was further immersed in 1 wt% aqueous methanol solution of ethyl bromide (manufactured by Wako Pure Chemical Industries, Ltd.) or pentyl bromide (manufactured by Wako Pure Chemical Industries, Ltd.), and the reaction was allowed to proceed at 35°C for 1 hour, and then at 50°C for 4 hours, thereby allowing modification of PEI covalently bound to the surface of the PET mesh with quaternary ammonium (quaternary-ammonium-modification step). The aqueous solution after the reaction was removed, and the mesh was washed with methanol and distilled water.

Finally, the mesh was immersed in an aqueous solution (pH = 4) of 0.75 wt% heparin sodium (manufactured by Organon API Inc.) and 0.1 mol/L sodium chloride, and the reaction was allowed to proceed at 70°C for 6 hours, thereby allowing ionic bonding with PEI (second coating step). The aqueous solution after the reaction was removed, and the mesh was washed with distilled water.

Here, a PET mesh subjected to the second coating step without performing the quaternary-ammonium-modification step was provided as Sample 1; a PET mesh subjected to the quaternary-ammonium-modification step using ethyl bromide was provided as Sample 2; and a PET mesh subjected to the quaternary-ammonium-modification step using pentyl bromide was provided as Sample 3.

As a result of analysis of the average thicknesses of the coating material by STEM, the average thicknesses of the coating materials in Samples 1 to 3 were found to be 31 nm, 26 nm, and 28 nm, respectively. Samples 1 to 3 were subjected to evaluation of their performances using the methods described in the later-mentioned Evaluations 1 to 8. The results are shown in Table 2.

(Example 2)

The same operation as in Example 1 was carried out except that PEI (average molecular weight, about 70,000; manufactured by Wako Pure Chemical Industries, Ltd.) was used in the first coating step.

Here, a PET mesh subjected to the second coating step without performing the quaternary-ammonium-modification step was provided as Sample 4; a PET mesh subjected to the quaternary-ammonium-modification step using ethyl bromide was provided as Sample 5; and a PET mesh subjected to the quaternary-ammonium-modification step using pentyl bromide was provided as Sample 6.

As a result of analysis of the average thicknesses of the coating material by STEM, the average thicknesses of the coating materials in Samples 4 to 6 were found to be 44 nm, 41 nm, and 39 nm, respectively. Samples 4 to 6 were subjected to evaluation of their performances using the methods described in the later-mentioned Evaluations 1 to 8. The results are shown in Table 2.

(Example 3)

The same operation as in Example 1 was carried out except that PEI (LUPASOL (registered trademark) P; manufactured by BASF)) was used in the first coating step.

Here, a PET mesh subjected to the second coating step without performing the quaternary-ammonium-modification step was provided as Sample 7; a PET mesh subjected to the quaternary-ammonium-modification step using ethyl bromide was provided as Sample 8; and a PET mesh subjected to the quaternary-ammonium-modification step using pentyl bromide was provided as Sample 9.

As a result of analysis of the average thicknesses of the coating material by STEM, the average thicknesses of the coating materials in Samples 7 to 9 were found to be 81 nm, 85 nm, and 79 nm, respectively. Samples 7 to 9 were subjected to evaluation of their performances using the methods described in the later-mentioned Evaluations 1 to 8. The results are shown in Table 2.

(Comparative Example 1)

The same operation as in the case of Sample 2 in Example 1 was carried out except that PEI (average molecular weight, about 600; manufactured by Wako Pure Chemical Industries, Ltd.) or PEI (LUPASOL (registered trademark) SK; manufactured by BASF) was used in the first coating step.

Here, a PET mesh subjected to the first coating step using PEI (average molecular weight, about 600; manufactured by Wako Pure Chemical Industries, Ltd.) was provided as Sample 10, and a PET mesh subjected to the first coating step using PEI (LUPASOL (registered trademark) SK, manufactured by BASF) was provided as Sample 11.

As a result of analysis of the average thicknesses of the coating material by STEM, the average thicknesses of the coating materials in Samples 10 and 11 were found to be not more than 10 nm, and to be 11 nm, respectively. Samples 10 and 11 were subjected to evaluation of their performances using the methods described in the later-mentioned Evaluations 1 to 8. The results are shown in Table 2.

(Comparative Example 2)

The first coating step was carried out by the same operation as in Example 3, and the PET mesh was then immersed in an aqueous solution of 0.5 wt% DMT-MM and 0.5 wt% PAA (manufactured by Wako Pure Chemical Industries, Ltd.), followed by allowing the reaction to proceed at 30°C for 2 hours (first additional step). The aqueous solution after the reaction was removed, and the mesh was washed with an aqueous sodium carbonate solution and distilled water.

The PET mesh was then immersed in an aqueous solution of 0.5 wt% DMT-MM and 5.0 wt% PEI, and the reaction was allowed to proceed at 30°C for 2 hours (second additional step). The aqueous solution after the reaction was removed, and the mesh was washed with distilled water. The quaternary-ammonium-modification step was carried out using ethyl bromide by the same operation as in Example 1, and the second coating step was then carried out.

Here, a PET mesh subjected to the second additional step using PEI (LUPASOL (registered trademark) P, manufactured by BASF) was provided as Sample 12.

As a result of analysis of the average thickness of the coating material by STEM, the average thickness of the coating material in Sample 12 was found to be 533 nm. Sample 12 was subjected to evaluation of its performances using the methods described in the later-mentioned Evaluations 1 to 8. The results are shown in Table 2.

(Comparative Example 3)

The first coating step was carried out by the same operation as in Example 3, and the PET mesh was then immersed in an aqueous solution of 0.5 wt% DMT-MM and 0.5 wt% PAA (manufactured by Wako Pure Chemical Industries, Ltd.), followed by allowing the reaction to proceed at 30°C for 2 hours (first additional step). The aqueous solution after the reaction was removed, and the mesh was washed with an aqueous sodium carbonate solution and distilled water.

The PET mesh was then immersed in an aqueous solution of 0.5 wt% DMT-MM and 5.0 wt% PEI, and the reaction was allowed to proceed at 30°C for 2 hours (second additional step). The aqueous solution after the reaction was removed, and the mesh was washed with distilled water.

The PET mesh was then further immersed in an aqueous solution of 0.5 wt% DMT-MM and 0.5 wt% PAA (Wako Pure Chemical Industries, Ltd.), and the reaction was allowed to proceed at 30°C for 2 hours (third additional step). The aqueous solution after the reaction was removed, and the mesh was washed with an aqueous sodium carbonate solution and distilled water.

The PET mesh was then immersed in an aqueous solution of 0.5 wt% DMT-MM and 5.0 wt% PEI, and the reaction was allowed to proceed at 30°C for 2 hours (fourth additional step). The aqueous solution after the reaction was removed, and the mesh was washed with distilled water. The quaternary-ammonium-modification step was carried out using ethyl bromide by the same operation as in Example 1, and the second coating step was then carried out.

Here, a PET mesh subjected to the second additional step and the fourth additional step using PEI (LUPASOL (registered trademark) P, manufactured by BASF) was provided as Sample 13.

As a result of analysis of the average thickness of the coating material by STEM, the average thickness of the coating material in Sample 13 was found to be 821 nm. Sample 13 was subjected to evaluation of its performances using the methods described in the later-mentioned Evaluations 1 to 8. The results are shown in Table 2.

(Comparative Example 4)

A PET mesh was immersed in an aqueous solution of 5% PEI, and irradiated with 5 kGy γ-ray (JS-8500 Cobalt 60 γ-ray irradiator, manufactured by Nordion International Inc.) to allow covalent bonding (first coating step). The aqueous solution after the reaction was removed, and the mesh was washed with Triton-X100 (manufactured by Sigma-Aldrich), physiological saline, and distilled water. The quaternary-ammonium-modification step was carried out using ethyl bromide by the same operation as in Example 1, and the second coating step was then carried out.

Here, a PET mesh subjected to the first coating step using PEI (average molecular weight, about 10,000; manufactured by Wako Pure Chemical Industries, Ltd.) was provided as Sample 14, and a PET mesh subjected to the first coating step using PEI (LUPASOL (registered trademark) P, manufactured by BASF) was provided as Sample 15.

As a result of analysis of the average thicknesses of the coating material by STEM, the average thicknesses of the coating materials in Samples 14 and 15 were found to be 13 nm and 14 nm, respectively. Samples 14 and 15 were subjected to evaluation of their performances using the methods described in the later-mentioned Evaluations 1 to 8. The results are shown in Table 2.

(Comparative Example 5)

A PET mesh was immersed in an aqueous solution of 5% PEI, and heated at 80°C for 2 hours, thereby covalently binding PEI to the PET mesh by aminolysis reaction (first coating step). The aqueous solution after the reaction was removed, and the mesh was washed with distilled water. The quaternary-ammonium-modification step was carried out using ethyl bromide by the same operation as in Example 1, and the second coating step was then carried out.

Here, a PET mesh subjected to the first coating step using PEI (average molecular weight, about 10,000; manufactured by Wako Pure Chemical Industries, Ltd.) was provided as Sample 16, and a PET mesh subjected to the first coating step using PEI (LUPASOL (registered trademark) P, manufactured by BASF) was provided as Sample 17.

As a result of analysis of the average thicknesses of the coating material by STEM, the average thicknesses of the coating materials in Samples 16 and 17 were both found to be not more than 10 nm. Samples 16 and 17 were subjected to evaluation of their performances using the methods described in the later-mentioned Evaluations 1 to 8. The results are shown in Table 2.

(Evaluation 1: Surface Amount Estimated Based on Anti-factor Xa Activity after Washing with Physiological Saline)

Referring to the operating procedure for the "Test Team (registered trademark) Heparin S" kit (manufactured by Sekisui Medical Co., Ltd.), a calibration curve was prepared. More specifically, 200 $\mu$L of a standard heparin solution was placed in an Eiken tube, and the tube was incubated at 37°C for 6 minutes. Thereafter, 200 $\mu$L of a factor Xa solution was added thereto, and the resulting mixture was mixed, followed by incubation at 37°C for 30 seconds. Thereafter, 200 $\mu$L of a substrate solution (S-2222 solution) prewarmed to 37°C was added thereto, and the resulting mixture was mixed, followed by incubation at 37°C for 3 minutes. Thereafter, 300 $\mu$L of a reaction stop solution was added thereto, and the resulting mixture was mixed. To a microplate, 300 $\mu$L of the mixture was placed, and the absorbance at 405 nm was measured using a microplate reader (MTP-300, manufactured by Corona Electric Co., Ltd.), followed by preparing a calibration curve according to the operating procedure for the Test Team (registered trademark) Heparin S kit. Subsequently, an antithrombogenic material (for example, PET mesh) coated with a coating material was cut into a piece having a size of 0.5 cm width and 0.5 cm length, and the piece was washed using physiological saline at 37°C for 30 minutes. Referring to the operating procedure for the "Test Team (registered trademark) Heparin S" kit (manufactured by Sekisui Medical Co., Ltd.), reaction of the sample was carried out. More specifically, the sample after washing with physiological saline was placed in an Eiken tube, and 20 $\mu$L of normal human plasma, 160 $\mu$L of a buffer, and 20 $\mu$L of an antithrombin III solution were added thereto, followed by mixing the resulting mixture and incubating the mixture at 37°C for 6 minutes. Thereafter, 200 $\mu$L of the factor Xa solution was added thereto, and the resulting mixture was mixed, followed by incubation at 37°C for 30 seconds. Thereafter, 200 $\mu$L of the substrate solution prewarmed to 37°C was added thereto, and the resulting mixture was mixed, followed by incubation at 37°C for 3 minutes. Thereafter, 300 $\mu$L of the reaction stop solution was added thereto, and the resulting mixture was mixed. The solution after stopping the reaction was aliquoted into Eppendorf tubes. The sample was removed in this process. To a microplate, 300 $\mu$L of the solution was placed, and the absorbance at 405 nm was measured using a microplate reader (MTP-300, manufactured by Corona Electric Co., Ltd.), followed by calculating the surface amount estimated based on the anti-factor Xa activity according to the operating procedure for Test Team Heparin S. The larger the surface amount, the better. The surface amount is preferably not less than 40 mIU/cm$^2$, more preferably not less than 50 mIU/cm$^2$, still more preferably not less than 60 mIU/cm$^2$. Further, as a result of evaluation of the performance of the antithrombogenic material coated with the coating material by an animal experiment, it was found that the surface amount after washing with physiological saline for 30 minutes is preferably not less than 40 mIU/cm$^2$. Accordingly, in cases where the surface amount estimated based on the anti-factor Xa activity after washing with physiological saline for 30 minutes was not less than 40 mIU/cm$^2$, the surface amount was evaluated as large, and rated as ($\bigcirc$), while in cases where the surface amount was less than 40 mIU/cm$^2$, the surface amount was evaluated as small, and rated as ($\times$).

(Evaluation 2: Surface Amount Estimated Based on Anti-factor Xa Activity after Washing with Human Blood Plasma)

An antithrombogenic material (for example, PET mesh) coated with a coating material was cut into a piece having a size of 1cm width and 1cm length, and the piece was washed using 500 µL of human blood plasma (manufactured by Cosmo Bio Co., Ltd.) at 37°C for 6 hours. Thereafter, according to the method in the above-mentioned Evaluation 1, the amount of the compound having anticoagulant activity remaining on the surface of the antithrombogenic material was calculated. As a result of evaluation of the performance of the antithrombogenic material coated with the coating material by an animal experiment, it was found that the residual surface amount is preferably less than 40 mIU/cm$^2$. Accordingly, in cases where the residual surface amount estimated based on the anti-factor Xa activity after washing with human blood plasma for 6 hours was less than 40 mIU/cm$^2$, it was rated as (○), while in cases where the residual surface amount was not less than 40 mIU/cm$^2$, it was rated as (×).

(Evaluation 3: Total Supported Amount Estimated Based on Anti-factor Xa Activity)

An antithrombogenic material (for example, PET mesh) coated with a coating material was cut into a piece having a size of 1cm width and 1cm length, and the piece was immersed in 500 µL of human blood plasma at 37°C for 24 hours to allow elution of the compound having anticoagulant activity. Using "Test Team (registered trademark) Heparin S" (manufactured by Sekisui Medical Co., Ltd.), the amount of the compound having anticoagulant activity eluted into the human blood plasma as estimated based on the anti-factor Xa activity was calculated. Subsequently, the PET mesh after the immersion in human blood plasma for 24 hours was cut into a piece having a size of 0.5 cm width and 0.5 cm length, and the piece was washed using physiological saline at 37°C for 30 minutes. Thereafter, according to the method in the above-mentioned Evaluation 1, the surface amount of the compound having anticoagulant activity remaining on the surface of the antithrombogenic material was calculated. The sum of the total elution amount and the surface amount remaining on the surface of the antithrombogenic material measured based on the anti-factor Xa activity was calculated as the total supported amount. In cases where the total supported amount is too large, the microstructure of the surface of the base material is destroyed, while in cases where the total supported amount is too small, the antithrombogenicity of interest is less likely to be obtained. Further, in cases where the total supported amount is too large, the cationic surface exposed after elution of heparin may promote thrombus formation. As a result of evaluation of the performance of the antithrombogenic material coated with the coating material by an animal experiment, it was found that the total supported amount is preferably 50 to 1000 mIU/cm$^2$. Accordingly, in cases where the total supported amount was 50 to 1000 mIU/cm$^2$, it was rated as (○), while in cases where the total supported amount was smaller than 50 mIU/cm$^2$ or larger than 1000 mIU/cm$^2$, it was rated as (×).

(Evaluation 4: Surface Analysis by XPS)

An antithrombogenic material (for example, PET mesh) coated with a coating material was cut into a piece having a size of 1cm width and 1cm length, and the ratio of the abundance of nitrogen atoms to the abundance of total atoms on the surface of the antithrombogenic material was calculated by carrying out XPS measurement under the following conditions.

[Measurement Conditions]

Apparatus: ESCALAB 220iXL (manufactured by VG Scientific)
Excitation X-ray: monochromatic AlK α1, 2 ray (1486.6 eV)
X-ray diameter: 1 mm
X-electron escape angle: 90° (the angle of the detector with respect to the surface of the antithrombogenic material)
The surface of the antithrombogenic material herein means the portion from the measurement surface to a depth of 10 nm as detected under the measurement conditions in XPS wherein the X-electron escape angle, that is, the angle of the detector with respect to the surface of the antithrombogenic material, is 90°.

(Evaluation 5: Tensile Test)

An antithrombogenic material (for example, PET mesh) coated with a coating material was cut into a piece having a size of 1cm width and 4cm length, and the maximum stress of the base material was evaluated by carrying out a tensile test under the following conditions.

[Measurement Conditions]

Apparatus: tensile tester Tensilon RTE-1210 (manufactured by Orientec Co., Ltd.)
Load cell: 500 N
Initial sample length: 10 mm
Pulling rate: 5 mm/minute

The maximum stress of the base material was calculated from the maximum load evaluated using the tensile tester and the cross-sectional area of the base material as shown by the following Equation 10.

$$\text{Maximum stress of the base material coated with the coating material (MPa)}$$
$$= \text{maximum load (N) / cross-sectional area of the base material coated with the}$$
$$\text{coating material (mm}^2) \dots \text{Equation 10}$$

The cross-sectional area of the base material was determined by measuring the number of fibers present within a width of 1 cm using a scanning electron microscope (manufactured by Hitachi High-Technologies Corporation), and then performing calculation as shown by the following Equation 11.

$$\text{Cross-sectional area of the base material coated with the coating material}$$
$$(\text{mm}^2) = 13.5 \times 13.5 \times 3.14 \times 80 \text{ (fibers) / 1,000,000} \dots \text{Equation 11}$$

In cases where the maximum stress is low, the base material becomes fragile, and therefore the performance of the medical equipment may be affected. Accordingly, in cases where the maximum stress of the polyester-based polymer base material coated with the coating material was not less than 350 MPa, it was rated as ($\bigcirc$), while in cases where the maximum stress was less than 350 MPa, it was rated as ($\times$).

(Evaluation 6: Analysis of Thickness by STEM)

Using an antithrombogenic material (for example, PET mesh) coated with a coating material, the average thickness of the coating material was analyzed. The thickness for which sulfur atoms derived from the coating material can be found was analyzed using the STEM-EDX method, and the thickness for which nitrogen atoms derived from the coating material can be found was analyzed using the STEM-EELS method. The average thickness herein means the average of the values obtained at at least three positions.

[Measurement Conditions]

Apparatus: field emission transmission electron microscope JEM-2100F (manufactured by JEOL Ltd.)
EELS detector: GIF Tridiem (manufactured by GATAN, Inc.)
EDX detector: JED-2300T (manufactured by JEOL Ltd.)
Image acquisition: Digital Micrograph (manufactured by GATAN, Inc.)
Sample preparation: ultrathin sectioning (suspension using a copper microgrid; use of an acrylic resin as an embedding resin)
Acceleration voltage: 200 kV
Beam diameter: 0.7-nm diameter
Energy resolution: about 1.0 eV FWHM

In cases where the thickness of the coating material is too large, the microstructure on the surface of the base material is destroyed, and therefore the performance of the medical equipment is affected. On the other hand, in cases where the coating material is too thin, the antithrombogenicity of interest cannot be obtained. Further, in cases where the thickness of the coating material is too large, the cationic polymer exposed after elution of the compound having anticoagulant activity may promote thrombus formation. As a result of evaluation of the performance of the antithrombogenic material coated with the coating material by an animal experiment, it was found that the average thickness of the coating material is preferably 15 to 400 nm. Accordingly, in cases where each of the average thickness for which sulfur atoms were found and the average thickness for which nitrogen atoms were found was 15 to 400 nm, the average thickness was rated as (○), while in cases where the average thickness for which either one kind of atoms were found was smaller than 15 nm or larger than 400 nm, the average thickness was rated as (×).

(Evaluation 7: Animal Experiment Using Free-thrombus Capture Device)

Using a PET mesh (diameter, 27 $\mu$m; interfiber distance, 100 $\mu$m) coated with a coating material, a free-thrombus capture device was prepared, and an animal experiment was carried out for evaluation of its antithrombogenicity. Under severe conditions where no heparin is administered, the free-thrombus capture device was placed in the common carotid artery of beagle dogs (male; body weight, 10 to 12 kg), and blood flow was observed by echo and a contrast medium. The average closing time at which the blood flow in the distal side of the free-thrombus capture device became unobservable due to formation of a thrombus on the PET mesh of the free-thrombus capture device was measured. The average closing time herein means the average of the values obtained by at least three replicates. In cases where the average closing time was not less than 20 minutes, the antithrombogenicity was evaluated as high, and rated as (○), while in cases where the average closing time was less than 20 minutes, the antithrombogenicity was evaluated as insufficient, and rated as (×). In general, at clinical sites, free-thrombus capture devices are used under conditions where an anticoagulant (such as heparin) is administered, and they are used in catheter treatment by a procedure which takes about 20 minutes. Thus, in cases where the closing time under severe conditions where no heparin is administered is not less than 20 minutes, clinical use of the free-thrombus capture device may be possible, so that the antithrombogenicity was rated as (○).

(Evaluation 8: Animal Experiment Using Artificial Blood Vessel)

Referring to a document by P. C. Begovac et al (Eur Vasc Endovasc Surg 25, 432-437 2003) and the like, a PET artificial blood vessel coated with a coating material (inner diameter, 3 mm; length, 3 cm) was transplanted to a dog carotid artery, and its antithrombogenicity and cellular adhesiveness were evaluated. From two days before the transplantation, oral administration of 100 mg/day of aspirin and 50 mg/day of dipyridamole was carried out. The administration was carried out every day until Day 28, when the artificial blood vessel was removed. After intravenous administration of 100 IU/kg of heparin and blocking of the blood flow with a clamp, the blood vessel was trimmed by 5 mm. The artificial blood vessel was transplanted to the common carotid artery by end-to-end suture. Thereafter, the patency of the artificial blood vessel was monitored by carrying out an echo test every seven days. As shown by the following Equation 12, the patency on Day 28 was calculated.

$$\text{Patency (\%)} = Nt \times 100 \,/\, \text{number of artificial blood vessels transplanted} \ldots$$

$$\text{Equation 12}$$

Nt: Number of artificial blood vessels that maintained patency until Day 28

The artificial blood vessels that maintained patency until Day 28 were subjected to histopathological tests for evaluation of the coverage with endothelial cells. As shown by the following Equation 13, the coverage with endothelial cells was calculated.

$$\text{Coverage with endothelial cells (\%)} = Lt \text{ (cm)} \times 100 \,/\, \text{total length of the}$$

$$\text{artificial blood vessel (cm)} \ldots \text{Equation 13}$$

Lt: Length for which endothelial cells immigrated (cm)

In cases where the average patency was not less than 60% on Day 28, and the average coverage with endothelial cells was not less than 40% one month later, the antithrombogenicity and the cellular adhesiveness were evaluated as high, and rated as (○). Otherwise, the antithrombogenicity or the cellular adhesiveness was evaluated as insufficient, and rated as (×).

[Table 2]

| | Sample | Cationic polymer | Weight average molecular weight of cationic polymer | Compound having sulfur element | Abundance ratio of nitrogen element (atomic percent) | Carbon number of alkyl group | Average thickness of coating material (nm) | Rating of anti-factor Xa activity after 30 minutes of washing with physiological saline | Rating of anti-factor Xa activity after 6 hours of washing with blood plasma | Rating of total supported amount estimated based on anti-factor Xa activity | Rating of maximum stress of base material coated with coating material Rating of antithrombogenicity by animal experiment using free-thrombus capture device | Rating of antithrombogenicity by animal experiment using free-thrombus capture device | Rating of antithrombogenicity and cellular adhesiveness by animal experiment using artificial blood vessel |
|---|---|---|---|---|---|---|---|---|---|---|---|---|---|
| Example 1 | 1 | Polyethyleneimine | 10000 | Heparin | 8.5 | 0 | 31 | ○ | ○ | ○ | ○ | ○ | ○ |
| | 2 | Polvethvleneimine | 10000 | Heparin | 8.4 | 2 | 26 | ○ | ○ | ○ | ○ | ○ | ○ |
| | 3 | Polyethyleneimine | 10000 | Heparin | 8.4 | 5 | 28 | ○ | ○ | ○ | ○ | ○ | ○ |
| Example 2 | 4 | Polyethyleneimine | 70000 | Heparin | 8.4 | 0 | 44 | ○ | ○ | ○ | ○ | ○ | ○ |
| | 5 | Polyethyleneimine | 70000 | Heparin | 8.3 | 2 | 41 | ○ | ○ | ○ | ○ | ○ | ○ |
| | 6 | Polyethyleneimine | 70000 | Heparin | 8.2 | 5 | 39 | ○ | ○ | ○ | ○ | ○ | ○ |
| Example 3 | 7 | Polyethyleneimine | 750000 | Heparin | 8.3 | 0 | 81 | ○ | ○ | ○ | ○ | ○ | ○ |
| | 8 | Polyethyleneimine | 750000 | Heparin | 8.2 | 2 | 85 | ○ | ○ | ○ | ○ | ○ | ○ |
| | 9 | Polyethyleneimine | 750000 | Heparin | 8.0 | 5 | 79 | ○ | ○ | ○ | ○ | ○ | ○ |
| Comparative Example 1 | 10 | Polyethyleneimine | 600 | Heparin | 3.4 | 2 | 10< | × | ○ | × | ○ | × | × |
| | 11 | Polyethyleneimine | 2000000 | Heparin | 4.2 | 2 | 11 | × | ○ | × | ○ | × | × |

| | Sample | Cationic polymer | Weight average molecular weight of cationic polymer | Compound having sulfur element | Abundance ratio of nitrogen element (atomic percent) | Carbon number of alkyl group | Average thickness of coating material (nm) | Rating of anti-factor Xa activity after 30 minutes of washing with physiological saline | Rating of anti-factor Xa activity after 6 hours of washing with blood plasma | Rating of total supported amount estimated based on anti-factor Xa activity | Rating of maximum stress of base material coated with coating material Rating of antithrombogenicity by animal experiment using free-thrombus capture device | Rating of antithrombogenicity by animal experiment using free-thrombus capture device | Rating of antithrombogenicity and cellular adhesiveness by animal experiment using artificial blood vessel |
|---|---|---|---|---|---|---|---|---|---|---|---|---|---|
| Comparative Example 2 | 12 | Polyethyleneimine | 750000 | Heparin | 9.8 | 2 | 533 | ○ | × | × | ○ | × | × |
| Comparative Example 3 | 13 | Polyethyleneimine | 750000 | Heparin | 12.8 | 2 | 821 | ○ | × | × | ○ | × | × |
| Comparative Example 4 | 14 | Polyethyleneimine | 10000 | Heparin | 6.0 | 2 | 13 | × | ○ | × | ○ | × | × |
| | 15 | Polyethyleneimine | 750000 | Heparin | 6.4 | 2 | 14 | × | ○ | × | ○ | × | × |
| Comparative Example 5 | 16 | Polyethyleneimine | 10000 | Heparin | 3.5 | 2 | 10< | × | ○ | × | ○ | × | × |
| | 17 | Polyethyleneimine | 750000 | Heparin | 3.4 | 2 | 10< | × | ○ | × | ○ | × | × |

EP 3 305 342 A1

26

(Example 4)

A PET mesh (diameter, 27 $\mu$m; interfiber distance, 100 $\mu$m) as a base material was immersed in an aqueous solution of 0.6 wt% potassium permanganate (manufactured by Wako Pure Chemical Industries, Ltd.) and 0.6 mol/L sulfuric acid (manufactured by Wako Pure Chemical Industries, Ltd.), and the reaction was allowed to proceed at 60°C for 24 hours, thereby hydrolyzing and oxidizing the PET mesh (hydrolysis/oxidation step). The aqueous solution after the reaction was removed, and the mesh was washed with hydrochloric acid (manufactured by Wako Pure Chemical Industries, Ltd.) and distilled water.

Subsequently, the PET mesh was immersed in an aqueous solution of 0.5 wt% DMT-MM (manufactured by Wako Pure Chemical Industries, Ltd.) and 5.0 wt% PEI (LUPASOL (registered trademark) P, manufactured by BASF; weight average molecular weight, 750,000), and the reaction was allowed to proceed at 50°C for 2 hours, thereby covalently binding PEI to the PET mesh by condensation reaction. The aqueous solution after the reaction was removed, and the mesh was washed with distilled water.

The PET mesh was further immersed in 1 wt% aqueous methanol solution of ethyl bromide (manufactured by Wako Pure Chemical Industries, Ltd.), and the reaction was allowed to proceed at 35°C for 1 hour, and then at 50°C for 4 hours, thereby allowing modification of PEI covalently bound to the surface of the PET mesh with quaternary ammonium (quaternary-ammonium-modification step). The aqueous solution after the reaction was removed, and the mesh was washed with methanol and distilled water.

The ratio of the abundance of positively charged functional groups to the abundance of negatively charged functional groups in the base material after the condensation reaction of PEI was 24. The ratio of the abundance of negatively charged functional groups in the base material after the covalent bonding of the polymer having a positively charged functional group to the abundance of negatively charged functional groups in the base material before the covalent bonding of the polymer having a positively charged functional group was 21%. The results are shown in Table 3.

Finally, the mesh was immersed in an aqueous solution (pH = 4) of 0.75 wt% heparin sodium (manufactured by Organon API Inc.) and 0.1 mol/L sodium chloride, and the reaction was allowed to proceed at 70°C for 6 hours, thereby allowing ionic bonding with PEI. The aqueous solution after the reaction was removed, and the mesh was washed with distilled water.

The obtained sample was subjected to measurement and evaluation of the surface amount based on the anti-factor Xa activity after 30 minutes of immersion in physiological saline (Evaluation 9). The results are shown in Table 3. As shown in Table 3, the surface amount evaluated based on the anti-factor Xa activity was 113 mIU/cm$^2$, and the sample therefore had antithrombogenicity. According to evaluation of the hemolytic toxicity (Evaluation 10), the sample was nonhemolytic (-). As shown in Table 3, in the animal experiment using a free-thrombus capture device (Evaluation 7), the sample was evaluated as having high antithrombogenicity (○), and, in the animal experiment using an artificial blood vessel (Evaluation 8), the sample was evaluated as having antithrombogenicity or high antithrombogenicity, as well as cellular adhesiveness (○).

(Example 5)

First, by the same operation as in Example 4, a PET mesh was hydrolyzed and oxidized. Subsequently, PEI was covalently bound to the base material by the same operation as in Example 4 except that the concentration of the aqueous PEI solution was 2.5 wt%, and the quaternary-ammonium-modification step was carried out using ethyl bromide, followed by ionically binding heparin to the PEI by the same operation as in Example 4.

The ratio of the abundance of positively charged functional groups to the abundance of negatively charged functional groups in the base material after the condensation reaction of PEI was 23. The ratio of the abundance of negatively charged functional groups in the base material after the covalent bonding of the polymer having a positively charged functional group to the abundance of negatively charged functional groups in the base material before the covalent bonding of the polymer having a positively charged functional group was 19%. The results are shown in Table 3.

The obtained sample was subjected to measurement and evaluation of the surface amount based on the anti-factor Xa activity after 30 minutes of immersion in physiological saline (Evaluation 9). The results are shown in Table 3. As shown in Table 3, the surface amount evaluated based on the anti-factor Xa activity was 103 mIU/cm$^2$, and the sample therefore had antithrombogenicity. According to evaluation of the hemolytic toxicity (Evaluation 10), the sample was nonhemolytic (-). As shown in Table 3, in the animal experiment using a free-thrombus capture device (Evaluation 7), the sample was evaluated as having high antithrombogenicity (○), and, in the animal experiment using an artificial blood vessel (Evaluation 8), the sample was evaluated as having antithrombogenicity or high antithrombogenicity, as well as cellular adhesiveness (○).

(Example 6)

First, by the same operation as in Example 4, a PET mesh was hydrolyzed and oxidized. Subsequently, PEI was covalently bound to the base material by the same operation as in Example 4 except that the concentration of the aqueous PEI solution was 1.5 wt%, and the quaternary-ammonium-modification step was carried out using ethyl bromide, followed by ionically binding heparin to the PEI by the same operation as in Example 4.

The ratio of the abundance of positively charged functional groups to the abundance of negatively charged functional groups in the base material after the condensation reaction of PEI was 15. The ratio of the abundance of negatively charged functional groups in the base material after the covalent bonding of the polymer having a positively charged functional group to the abundance of negatively charged functional groups in the base material before the covalent bonding of the polymer having a positively charged functional group was 21%. The results are shown in Table 3.

The obtained sample was subjected to measurement and evaluation of the surface amount based on the anti-factor Xa activity after 30 minutes of immersion in physiological saline (Evaluation 9). The results are shown in Table 3. As shown in Table 3, the surface amount evaluated based on the anti-factor Xa activity was 75 mIU/cm$^2$, and the sample therefore had antithrombogenicity. According to evaluation of the hemolytic toxicity (Evaluation 10), the sample was nonhemolytic (-). As shown in Table 3, in the animal experiment using a free-thrombus capture device (Evaluation 7), the sample was evaluated as having high antithrombogenicity ($\bigcirc$), and, in the animal experiment using an artificial blood vessel (Evaluation 8), the sample was evaluated as having antithrombogenicity or high antithrombogenicity, as well as cellular adhesiveness ($\bigcirc$).

(Example 7)

First, the hydrolysis/oxidation step was carried out by the same operation as in Example 4 except that the concentration of the aqueous sulfuric acid solution was 0.1 mol/L. Subsequently, PEI was covalently bound to the base material by the same operation as in Example 4 using 0.5 wt% DMT-MM and 5.0 wt% PEI (LUPASOL (registered trademark) P, manufactured by BASF; weight average molecular weight, 750,000), and the quaternary-ammonium-modification step was carried out using ethyl bromide, followed by ionically binding heparin to the PEI by the same operation as in Example 4.

The ratio of the abundance of positively charged functional groups to the abundance of negatively charged functional groups in the base material after the condensation reaction of PEI was 20. The ratio of the abundance of negatively charged functional groups in the base material after the covalent bonding of the polymer having a positively charged functional group to the abundance of negatively charged functional groups in the base material before the covalent bonding of the polymer having a positively charged functional group was 19%. The results are shown in Table 3.

The obtained sample was subjected to measurement and evaluation of the surface amount based on the anti-factor Xa activity after 30 minutes of immersion in physiological saline. The results are shown in Table 3. As shown in Table 3, the surface amount evaluated based on the anti-factor Xa activity was 100 mIU/cm$^2$, and the sample therefore had antithrombogenicity. According to evaluation of the hemolytic toxicity (Evaluation 10), the sample was nonhemolytic (-). As shown in Table 3, in the animal experiment using a free-thrombus capture device (Evaluation 7), the sample was evaluated as having high antithrombogenicity ($\bigcirc$), and, in the animal experiment using an artificial blood vessel (Evaluation 8), the sample was evaluated as having antithrombogenicity or high antithrombogenicity, as well as cellular adhesiveness ($\bigcirc$).

(Example 8)

First, by the same operation as in Example 7, a PET mesh was hydrolyzed and oxidized. Subsequently, PEI was covalently bound to the base material by the same operation as in Example 4 except that the concentration of the aqueous PEI solution was 2.0 wt%, and the quaternary-ammonium-modification step was carried out using ethyl bromide, followed by ionically binding heparin to the PEI by the same operation as in Example 4.

The ratio of the abundance of positively charged functional groups to the abundance of negatively charged functional groups in the base material after the condensation reaction of PEI was 12. The ratio of the abundance of negatively charged functional groups in the base material after the covalent bonding of the polymer having a positively charged functional group to the abundance of negatively charged functional groups in the base material before the covalent bonding of the polymer having a positively charged functional group was 20%. The results are shown in Table 3.

The obtained sample was subjected to measurement and evaluation of the surface amount based on the anti-factor Xa activity after 30 minutes of immersion in physiological saline. The results are shown in Table 3. As shown in Table 3, the surface amount evaluated based on the anti-factor Xa activity was 97 mIU/cm$^2$, and the sample therefore had antithrombogenicity. According to evaluation of the hemolytic toxicity (Evaluation 10), the sample was nonhemolytic (-). As shown in Table 3, in the animal experiment using a free-thrombus capture device (Evaluation 7), the sample was evaluated as having high antithrombogenicity (○), and, in the animal experiment using an artificial blood vessel (Evaluation 8), the sample was evaluated as having antithrombogenicity or high antithrombogenicity, as well as cellular adhesiveness (○).

(Example 9)

First, the step of hydrolysis and oxidation of the PET mesh was carried out by the same operation as in Example 4 except that the concentration of potassium permanganate was 3.0 wt%; the concentration of the aqueous sulfuric acid solution was 0.1 mol/L; and the reaction time was 3 hours. Subsequently, PEI was covalently bound to the base material by the same operation as in Example 4 using 0.5 wt% DMT-MM and 5.0 wt% PEI (manufactured by Wako Pure Chemical Industries, Ltd.; weight average molecular weight, 10,000), and the quaternary-ammonium-modification step was carried out using ethyl bromide, followed by ionically binding heparin to the PEI by the same operation as in Example 4.

The ratio of the abundance of positively charged functional groups to the abundance of negatively charged functional groups in the base material after the condensation reaction of PEI was 10. The ratio of the abundance of negatively charged functional groups in the base material after the covalent bonding of the polymer having a positively charged functional group to the abundance of negatively charged functional groups in the base material before the covalent bonding of the polymer having a positively charged functional group was 24%. The results are shown in Table 3.

The obtained sample was subjected to measurement and evaluation of the surface amount based on the anti-factor Xa activity after 30 minutes of immersion in physiological saline. The results are shown in Table 3. As shown in Table 3, the surface amount evaluated based on the anti-factor Xa activity was 71 $mIU/cm^2$, and the sample therefore had antithrombogenicity. According to evaluation of the hemolytic toxicity (Evaluation 10), the sample was nonhemolytic (-). As shown in Table 3, in the animal experiment using a free-thrombus capture device (Evaluation 7), the sample was evaluated as having high antithrombogenicity (○), and, in the animal experiment using an artificial blood vessel (Evaluation 8), the sample was evaluated as having antithrombogenicity or high antithrombogenicity, as well as cellular adhesiveness (○).

(Comparative Example 6)

First, hydrolysis and oxidation of the PET mesh was carried out by the same operation as in Example 9, and PEI was covalently bound to the base material using 0.5 wt% DMT-MM (manufactured by Wako Pure Chemical Industries, Ltd.) and 5.0 wt% PEI (LUPASOL (registered trademark) P, manufactured by BASF; weight average molecular weight, 750,000).

After the covalent bonding of the PEI to the base material, the PET mesh was then immersed in 0.5 wt% DMT-MM and 40 wt% succinic anhydride (manufactured by Wako Pure Chemical Industries, Ltd.) in dimethylacetamide, and the reaction was allowed to proceed at 50°C for 17 hours (first additional step). The solution after the reaction was removed, and the mesh was washed with methanol and distilled water. The PET mesh was then immersed in an aqueous solution of 0.5 wt% DMT-MM and 5.0 wt% PEI, and the reaction was allowed to proceed at 30°C for 2 hours (second additional step). The aqueous solution after the reaction was removed, and the mesh was washed with distilled water. The quaternary-ammonium-modification step was carried out using ethyl bromide by the same operation as in Example 4, and then heparin was ionically bound to the PEI by the same operation as in Example 4.

The ratio of the abundance of positively charged functional groups to the abundance of negatively charged functional groups in the base material after the condensation reaction of PEI was 40. The ratio of the abundance of negatively charged functional groups in the base material after the covalent bonding of the polymer having a positively charged functional group to the abundance of negatively charged functional groups in the base material before the covalent bonding of the polymer having a positively charged functional group was 5%. The results are shown in Table 3.

The obtained sample was subjected to measurement and evaluation of the surface amount based on the anti-factor Xa activity after 30 minutes of immersion in physiological saline (Evaluation 9). The results are shown in Table 3. As shown in Table 3, the surface amount evaluated based on the anti-factor Xa activity was 181 $mIU/cm^2$, and the sample therefore had antithrombogenicity. According to evaluation of the hemolytic toxicity (Evaluation 10), the sample was nonhemolytic (-). As shown in Table 3, in the animal experiment using a free-thrombus capture device (Evaluation 7), the antithrombogenicity was insufficient (×), and, in the animal experiment using an artificial blood vessel (Evaluation 8), the antithrombogenicity or the cellular adhesiveness was insufficient (×).

(Comparative Example 7)

After covalently binding PEI to the base material by the same operation as in Comparative Example 6, the PET mesh was immersed in an aqueous solution of 0.5 wt% DMT-MM and 0.5 wt% PAA (manufactured by Wako Pure Chemical Industries, Ltd.; weight average molecular weight, 5000), and the reaction was allowed to proceed at 30°C for 2 hours (first additional step). The aqueous solution after the reaction was removed, and the mesh was washed with an aqueous sodium carbonate solution and distilled water.

The PET mesh was then immersed in an aqueous solution of 0.5 wt% DMT-MM and 5.0 wt% PEI (LUPASOL (registered trademark) P, manufactured by BASF; weight average molecular weight, 750,000), and the reaction was allowed to proceed at 30°C for 2 hours (second additional step). The aqueous solution after the reaction was removed, and the mesh was washed with distilled water. The quaternary-ammonium-modification step was carried out using ethyl bromide by the same operation as in Example 4, and then heparin was ionically bound to the PEI by the same operation as in Example 4.

The ratio of the abundance of positively charged functional groups to the abundance of negatively charged functional groups in the base material after the condensation reaction of PEI was 121. The ratio of the abundance of negatively charged functional groups in the base material after the covalent bonding of the polymer having a positively charged functional group to the abundance of negatively charged functional groups in the base material before the covalent bonding of the polymer having a positively charged functional group was 5%.

The obtained sample was subjected to measurement and evaluation of the surface amount based on the anti-factor Xa activity after 30 minutes of immersion in physiological saline (Evaluation 9). The results are shown in Table 3. As shown in Table 3, the surface amount evaluated based on the anti-factor Xa activity was 173 mIU/cm$^2$, and the sample therefore had antithrombogenicity. According to evaluation of the hemolytic toxicity (Evaluation 10), the sample was nonhemolytic (-). As shown in Table 3, in the animal experiment using a free-thrombus capture device (Evaluation 7), the antithrombogenicity was insufficient ($\times$), and, in the animal experiment using an artificial blood vessel (Evaluation 8), the antithrombogenicity or the cellular adhesiveness was insufficient ($\times$).

(Comparative Example 8)

The first additional step was carried out by the same operation as in Comparative Example 7 except that the weight average molecular weight of PAA was 1,000,000. Subsequently, the second additional step using PEI and the quaternary-ammonium-modification step using ethyl bromide were carried out by the same operation as in Comparative Example 7, and then heparin was ionically bound to the PEI by the same operation as in Example 4.

The ratio of the abundance of positively charged functional groups to the abundance of negatively charged functional groups in the base material after the condensation reaction of PEI was 180. The ratio of the abundance of negatively charged functional groups in the base material after the covalent bonding of the polymer having a positively charged functional group to the abundance of negatively charged functional groups in the base material before the covalent bonding of the polymer having a positively charged functional group was 2%. The results are shown in Table 3.

The obtained sample was subjected to measurement and evaluation of the surface amount based on the anti-factor Xa activity after 30 minutes of immersion in physiological saline (Evaluation 9). The results are shown in Table 3. As shown in Table 3, the surface amount evaluated based on the anti-factor Xa activity was 181 mIU/cm$^2$, and the sample therefore had antithrombogenicity. According to evaluation of the hemolytic toxicity (Evaluation 10), the sample was nonhemolytic (-). As shown in Table 3, in the animal experiment using a free-thrombus capture device (Evaluation 7), the antithrombogenicity was insufficient ($\times$), and, in the animal experiment using an artificial blood vessel (Evaluation 8), the antithrombogenicity or the cellular adhesiveness was insufficient ($\times$).

(Comparative Example 9)

First, after hydrolysis and oxidation of the PET mesh by the same operation as in Example 7, PEI was covalently bound to the base material using 0.5 wt% DMT-MM and 0.5 wt% PEI (LUPASOL (registered trademark) P, manufactured by BASF; weight average molecular weight, 750,000), and the quaternary-ammonium-modification step was carried out using ethyl bromide, followed by ionically binding heparin to the PEI by the same operation as in Example 3.

The ratio of the abundance of positively charged functional groups to the abundance of negatively charged functional groups in the base material after the condensation reaction of PEI was 6. The ratio of the abundance of negatively charged functional groups in the base material after the covalent bonding of the polymer having a positively charged functional group to the abundance of negatively charged functional groups in the base material before the covalent bonding of the polymer having a positively charged functional group was 19%. The results are shown in Table 3.

The obtained sample was subjected to measurement and evaluation of the surface amount based on the anti-factor Xa activity after 30 minutes of immersion in physiological saline (Evaluation 9). The results are shown in Table 3. As shown in Table 3, the surface amount evaluated based on the anti-factor Xa activity was 38 mIU/cm$^2$, and the sample was therefore shown to have no antithrombogenicity. According to evaluation of the hemolytic toxicity (Evaluation 10), the sample was nonhemolytic (-). As shown in Table 3, in the animal experiment using a free-thrombus capture device (Evaluation 7), the antithrombogenicity was insufficient ($\times$), and, in the animal experiment using an artificial blood vessel (Evaluation 8), the antithrombogenicity or the cellular adhesiveness was insufficient ($\times$).

(Comparative Example 10)

After hydrolysis and oxidation of the PET mesh by the same operation as in Example 9, PEI was ionically bound to the base material without addition of DMT-MM, and the quaternary-ammonium-modification step was carried out using ethyl bromide, followed by ionically binding heparin to the PEI by the same operation as in Example 4.

The ratio of the abundance of positively charged functional groups to the abundance of negatively charged functional groups in the base material after the condensation reaction of PEI was 9. The ratio of the abundance of negatively charged functional groups in the base material after the covalent bonding of the polymer having a positively charged functional group to the abundance of negatively charged functional groups in the base material before the covalent bonding of the polymer having a positively charged functional group was 26%. The results are shown in Table 3.

The obtained sample was subjected to measurement and evaluation of the surface amount based on the anti-factor Xa activity after 30 minutes of immersion in physiological saline (Evaluation 9). The results are shown in Table 3. As shown in Table 3, the surface amount evaluated based on the anti-factor Xa activity was 36 mIU/cm$^2$, and the sample was therefore shown to have no antithrombogenicity. According to evaluation of the hemolytic toxicity (Evaluation 10), the sample was nonhemolytic (-). As shown in Table 3, in the animal experiment using a free-thrombus capture device (Evaluation 7), the antithrombogenicity was insufficient ($\times$), and, in the animal experiment using an artificial blood vessel (Evaluation 8), the antithrombogenicity or the cellular adhesiveness was insufficient ($\times$).

(Comparative Example 11)

After hydrolysis and oxidation of the PET mesh by the same operation as in Example 9, PEI was covalently bound to the base material using 0.5 wt% DMT-MM and 0.5 wt% PEI (manufactured by Wako Pure Chemical Industries, Ltd.; weight average molecular weight, 600), and the quaternary-ammonium-modification step was carried out using ethyl bromide, followed by ionically binding heparin to the PEI by the same operation as in Example 4.

The ratio of the abundance of positively charged functional groups to the abundance of negatively charged functional groups in the base material after the condensation reaction of PEI was 7. The ratio of the abundance of negatively charged functional groups in the base material after the covalent bonding of the polymer having a positively charged functional group to the abundance of negatively charged functional groups in the base material before the covalent bonding of the polymer having a positively charged functional group was 26%. The results are shown in Table 3.

The obtained sample was subjected to measurement and evaluation of the surface amount based on the anti-factor Xa activity after 30 minutes of immersion in physiological saline (Evaluation 9). The results are shown in Table 3. As shown in Table 3, the surface amount evaluated based on the anti-factor Xa activity was 34 mIU/cm$^2$, and the sample was therefore shown to have no antithrombogenicity. According to evaluation of the hemolytic toxicity (Evaluation 10), the sample was nonhemolytic (-). As shown in Table 3, in the animal experiment using a free-thrombus capture device (Evaluation 7), the antithrombogenicity was insufficient ($\times$), and, in the animal experiment using an artificial blood vessel (Evaluation 8), the antithrombogenicity or the cellular adhesiveness was insufficient ($\times$).

(Comparative Example 12)

After hydrolysis and oxidation of the PET mesh by the same operation as in Example 9, PEI was covalently bound to the base material using 0.5 wt% DMT-MM and 0.5 wt% PEI (manufactured by Wako Pure Chemical Industries, Ltd.; weight average molecular weight, 2,000,000), and the quaternary-ammonium-modification step was carried out using ethyl bromide, followed by ionically binding heparin to the PEI by the same operation as in Example 4.

The ratio of the abundance of positively charged functional groups to the abundance of negatively charged functional groups in the base material after the condensation reaction of PEI was 5. The ratio of the abundance of negatively charged functional groups in the base material after the covalent bonding of the polymer having a positively charged functional group to the abundance of negatively charged functional groups in the base material before the covalent bonding of the polymer having a positively charged functional group was 31%. The results are shown in Table 3.

The obtained sample was subjected to measurement and evaluation of the surface amount based on the anti-factor Xa activity after 30 minutes of immersion in physiological saline (Evaluation 9). The results are shown in Table 3. As shown in Table 3, the surface amount evaluated based on the anti-factor Xa activity was 28 mIU/cm$^2$, and the sample was therefore shown to have no antithrombogenicity. According to evaluation of the hemolytic toxicity (Evaluation 10), the sample was nonhemolytic (-). As shown in Table 3, in the animal experiment using a free-thrombus capture device (Evaluation 7), the antithrombogenicity was insufficient ($\times$), and, in the animal experiment using an artificial blood vessel (Evaluation 8), the antithrombogenicity or the cellular adhesiveness was insufficient ($\times$).

(Comparative Example 13)

The second additional step using PEI was carried out by the same operation as in Comparative Example 8, and then the third additional step was carried out again using PAA having a weight average molecular weight of 1,000,000, to provide a base material. The fourth additional step using PEI and the quaternary-ammonium-modification step using ethyl bromide were carried out by the same operation as in Example 9, and then heparin was ionically bound to the PEI by the same operation as in Example 4.

The ratio of the abundance of positively charged functional groups to the abundance of negatively charged functional groups in the base material after the condensation reaction of PEI was 244. The ratio of the abundance of negatively charged functional groups in the base material after the covalent bonding of the polymer having a positively charged functional group to the abundance of negatively charged functional groups in the base material before the covalent bonding of the polymer having a positively charged functional group was 2%. The results are shown in Table 3.

The obtained sample was subjected to measurement and evaluation of the surface amount based on the anti-factor Xa activity after 30 minutes of immersion in physiological saline (Evaluation 9). The results are shown in Table 3. As shown in Table 3, the surface amount evaluated based on the anti-factor Xa activity was 160 mIU/cm$^2$. However, since the ratio of the abundance of positively charged functional groups to the abundance of negatively charged functional groups in the base material was high, the sample was evaluated as mildly hemolytic (+) according to evaluation of the hemolytic toxicity (Evaluation 10). As shown in Table 3, in the animal experiment using a free-thrombus capture device (Evaluation 7), the antithrombogenicity was insufficient ($\times$), and, in the animal experiment using an artificial blood vessel (Evaluation 8), the antithrombogenicity or the cellular adhesiveness was insufficient ($\times$).

In relation to the antithrombogenicity of the material of the present invention, the method for evaluation of the surface amount based on the anti-factor Xa activity and the method for evaluation of the hemolytic toxicity are described below.

(Evaluation 9: Surface Amount Estimated Based on Anti-factor Xa Activity)

An antithrombogenic material (for example, PET mesh) was cut into a piece having a size of 0.5 cm × 0.5 cm, and the piece was washed with physiological saline at 37°C for 30 minutes. The washed PET mesh was reacted according to the procedure for "Test Team (registered trademark) Heparin S" (manufactured by Sekisui Medical Co., Ltd.), and the absorbance at 405 nm was measured using a microplate reader (MTP-300, manufactured by Corona Electric Co., Ltd.), followed by calculating the surface amount based on the anti-factor Xa activity according to the procedure for Test Team Heparin S. The higher the surface amount, the better. The surface amount is preferably not less than 40 mIU/cm$^2$, more preferably not less than 80 mIU/cm$^2$.

(Evaluation 10: Evaluation of Hemolytic Toxicity)

Fresh human blood was fed into an Erlenmeyer flask containing glass beads, such that the blood flowed along the wall surface of the flask. The flask was then placed on a palm, and horizontally shaken in a circular motion at a rate of about two rotations per second for about 5 minutes, to prepare defibrinated blood. An antithrombogenic material (for example, PET mesh) was cut into a piece having a size of 1.0 × 2.0 cm, and the piece was washed with physiological saline at 37°C for 30 minutes. The washed piece was placed in a 2-mL microtube. To the microtube containing the mesh, 1 mL of the defibrinated blood after 50-fold dilution with physiological saline was added, and the tube was then incubated at 37°C for 4 hours. Thereafter, the microtube was centrifuged at 750 G for 5 minutes. The resulting supernatant was collected, and subjected to measurement of the UV absorbance at 576 nm. In cases where the value calculated according to the Equation 9 was larger than 2, that is, in cases where the material was hemolytic, the material was rated as (+), while in cases where the value was not more than 2, that is, in cases where the material was nonhemolytic, the material was rated as (-). Since the material preferably has no hemolytic toxicity, the material is preferably nonhemolytic.a

[Table 3]

| | $Q_{ratio}$ (-) | $Q^-$ residue (%) | Surface amount of heparin estimated based on anti-factor Xa activity (MIU/cm$^2$) | Rating of antithrombogenicity by animal experiment using free-thrombus capture device | Rating of antithrombogenicity and cellular adhesiveness by animal experiment using artificial blood vessel | Evaluation of hemolytic toxicity |
|---|---|---|---|---|---|---|
| Example 4 | 24 | 21 | 113 | ○ | ○ | - |
| Example 5 | 23 | 19 | 103 | ○ | ○ | - |
| Example 6 | 15 | 21 | 75 | ○ | ○ | - |
| Example 7 | 20 | 19 | 100 | ○ | ○ | - |
| Example 8 | 12 | 2U | 97 | ○ | ○ | - |
| Example 9 | 10 | 24 | 71 | ○ | ○ | - |
| Comparative Example 6 | 40 | 5 | 181 | × | × | - |
| Comparative Example 7 | 121 | 5 | 173 | × | × | - |
| Comparative Example 8 | 180 | 2 | 181 | × | × | - |
| Comparative Example 9 | 6 | 19 | 38 | × | × | - |
| Comparative Example 10 | 9 | 26 | 36 | × | × | - |
| Comparative Example 11 | 7 | 26 | 34 | × | × | - |
| Comparative Example 12 | 5 | 31 | 28 | × | × | - |
| Comparative Example 13 | 244 | 2 | 160 | × | × | - |

INDUSTRIAL APPLICABILITY

The antithrombogenic material of the present invention can be used for medical tools (medical equipment and medical instruments) requiring maintenance of high antithrombogenicity for a long period, in the field of medicine.

**Claims**

1.  An antithrombogenic material comprising:

a coating material containing: a cationic polymer containing, as a constituent monomer, a compound selected from the group consisting of alkyleneimines, vinylamines, allylamines, lysine, protamine, and diallyldimethylammonium chloride; and an anionic compound containing a sulfur atom and having anticoagulant activity; and a base material whose surface is coated with said coating material;
wherein
said cationic polymer is covalently bound to said base material;
said anionic compound containing a sulfur atom and having anticoagulant activity is ionically bound to said cationic polymer; and

the average thickness of said coating material is 15 to 400 nm.

2. The antithrombogenic material according to claim 1, wherein the total amount of said anionic compound having anticoagulant activity contained in said coating material as calculated from anti-factor Xa activity is 50 to 1000 mIU/cm$^2$.

3. The antithrombogenic material according to claim 1 or 2, wherein the abundance ratio of nitrogen atoms to the abundance of total atoms as measured by X-ray photoelectron spectroscopy (XPS) on the surface is 6.5 to 9.5 atomic percent.

4. The antithrombogenic material according to any one of claims 1 to 3, wherein the weight average molecular weight of said cationic polymer is 10,000 to 1,000,000.

5. The antithrombogenic material according to any one of claims 1 to 4, wherein said base material is a polyester-based polymer, and the maximum stress of said antithrombogenic material is not less than 350 MPa.

6. The antithrombogenic material according to any one of claims 1 to 5, wherein
said cationic polymer has a positively charged functional group, and said base material has a negatively charged functional group; and
the abundance ratio of said positively charged functional group to said negatively charged functional group is 8.0 to 30.

7. The antithrombogenic material according to any one of claims 1 to 6, wherein the ratio of the abundance of said negatively charged functional group in said base material after the covalent bonding of said cationic polymer to the abundance of said negatively charged functional group in said base material before the covalent bonding of said cationic polymer is not more than 25%.

8. The antithrombogenic material according to any one of claims 1 to 7, having cellular adhesiveness.

9. A medical tool comprising the antithrombogenic material according to any one of claims 1 to 8.

## INTERNATIONAL SEARCH REPORT

| International application No. |
|---|
| PCT/JP2016/065665 |

A. CLASSIFICATION OF SUBJECT MATTER
*A61L33/00*(2006.01)i

According to International Patent Classification (IPC) or to both national classification and IPC

B. FIELDS SEARCHED

Minimum documentation searched (classification system followed by classification symbols)
A61L33/00

Documentation searched other than minimum documentation to the extent that such documents are included in the fields searched

| Jitsuyo Shinan Koho | 1922–1996 | Jitsuyo Shinan Toroku Koho | 1996–2016 |
| Kokai Jitsuyo Shinan Koho | 1971–2016 | Toroku Jitsuyo Shinan Koho | 1994–2016 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | WO 2014/168198 A1 (Toray Industries, Inc.), 16 October 2014 (16.10.2014), claims; paragraphs [0011], [0015], [0032] to [0037]; examples & US 2016/0067065 A1 claims; paragraphs [0010], [0014], [0040] to [0049]; examples & EP 2985042 A | 1–9 |
| Y | WO 2009/119761 A1 (Kaneka Corp.), 01 October 2009 (01.10.2009), claim 6; paragraphs [0005], [0031], [0034]; examples & US 2011/0084019 A1 claim 6; paragraphs [0007], [0043], [0046] & EP 2258416 A1 | 1–9 |

[×] Further documents are listed in the continuation of Box C.    [ ] See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 01 August 2016 (01.08.16) | 09 August 2016 (09.08.16) |

| Name and mailing address of the ISA/ | Authorized officer |
|---|---|
| Japan Patent Office 3-4-3,Kasumigaseki,Chiyoda-ku, Tokyo 100-8915,Japan | Telephone No. |

Form PCT/ISA/210 (second sheet) (January 2015)

35

**INTERNATIONAL SEARCH REPORT**

International application No.

PCT/JP2016/065665

C (Continuation). DOCUMENTS CONSIDERED TO BE RELEVANT

| Category* | Citation of document, with indication, where appropriate, of the relevant passages | Relevant to claim No. |
|---|---|---|
| A | JP 9-276394 A (Vascular Graft Research Center Co., Ltd.), 28 October 1997 (28.10.1997), entire text & US 6053939 A & EP 790042 A2 | 1-9 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4152075 B
- JP 3497612 B
- JP 10513074 W
- JP 60041947 B
- JP 60047287 B
- WO 2014168198 A
- JP 4273965 B
- JP 10151192 A
- JP 3341503 B
- JP 3834602 B
- JP 4982752 B

**Non-patent literature cited in the description**

- **P. C. BEGOVAC et al.** *Eur Vasc Endovasc Surg,* 2003, vol. 25, 432-437